# EUROPEAN PATENT APPLICATION

(11) **EP 4 752 233 A1**
(43) Date of publication of application: **03.06.2026**
(21) Application number: 24216264.2
(22) Date of filing: 28.11.2024
(51) Int. Cl.: C12Q 1/6886, G01N 33/574, G16B 25/10

(54) **PREDICTING EFFECTIVENESS OF ANDROGEN DEPRIVATION THERAPY IN PROSTATE CANCER**

(71) Applicant: Koninklijke Philips N.V., 5656 AE Eindhoven (NL)
(72) Inventor: HOFFMANN, Ralf Dieter, Eindhoven (NL)
(74) Representative: Philips Intellectual Property & Standards

(57) **Abstract**

The invention relates to methods for predicting an outcome for a prostate cancer patient. In particular the method describes a model based on the expression levels of marker genes which can be used to predict the effectiveness of androgen deprivation therapy (ADT). The method allows for treatment suggestions, particularly for those patients where ADT is predicted not to be effective, and alternative treatment may be desirable. The invention further describes kits useful for use in the described methods, as well as composition for use in treatment of prostate cancer based on a treatment outcome prediction described herein.

## Description

### FIELD OF THE INVENTION

The invention relates to methods for predicting an outcome for a prostate cancer patient. In particular the method describes a model based on the expression levels of marker genes which can be used to predict the effectiveness of androgen deprivation therapy (ADT). The method allows for treatment suggestions, particularly for those patients where ADT is predicted not to be effective, and alternative treatment may be desirable. The invention further describes kits useful for use in the described methods, as well as composition for use in treatment of prostate cancer based on a treatment outcome prediction described herein.

### BACKGROUND OF THE INVENTION

The number of new prostate cancer cases worldwide in 2020 is estimated at 1.4 million (Bergengren et al., 2023), while projection of estimated incidence in 2030 is likely to increase in developed countries (Cai et al., 2020). Despite significant research efforts, there is still a critical need for the identification of biomarkers in prostate cancer patients. Prognostic biomarkers are particularly crucial as they enable clinicians to better assess the risk associated with the disease, allowing for more tailored treatment strategies and allocation of resources. Early prostate cancer causes no symptoms (Sevcenco et al., 2019). Prostate cancer affects middle-aged men between the ages of 45 and 60 and is the highest cause of cancer associated mortalities in the Western countries (Sekhoacha et al., 2022). The risk factors related to prostate cancer include family risk, ethnicity, age, obesity, and other environmental factors. Prostate cancer is a heterogeneous disease both on the basis of epidemiology and genetics.

Despite significant research efforts, there is still a critical need for the identification of biomarkers in prostate cancer patients. Prognostic biomarkers are particularly crucial as they enable clinicians to better assess the risk associated with the disease, allowing for more tailored treatment strategies and allocation of resources. This personalized approach not only facilitates early intervention when necessary but also helps to avoid unnecessary treatments, ultimately leading to improved patient outcomes and quality of life. By leveraging biomarkers, clinicians can optimize the management of prostate cancer, enhancing both diagnosis and treatment decisions for individual patients.

WO2019122037A1 describes a method of pre-surgical risk stratification of a prostate cancer subject, comprising determining a gene expression profile for phosphodiesterase 4D variant 7 (PDE4D7) in a biological sample obtained from the subject, determining an expression based risk score for the subject based on the gene expression profile, and determining a pre-surgical prognostic risk score for the subject based on the expression based risk score and pre-surgical clinical variables of the subject.

In patients who undergo treatment, the most important clinical prognostic indicators of disease outcome are the stage, pretherapy PSA level, and Gleason score. The higher the grade and the stage, the poorer the prognosis, however a subset of patients in the poor prognosis group can be successfully treated. Therefore improved methods for predicting a patient outcome, particularly for those patients with poor indicators, are needed. In addition, suitable treatment options are needed for the predicted outcomes.

These drawbacks, among others, are overcome by the products and methods as outlined in the appended claims.

### SUMMARY OF THE INVENTION

In a first aspect the invention relates to a computer implemented method for predicting a treatment response of a subject suffering from prostate cancer to androgen deprivation therapy (ADT), the method comprising: receiving values representing the expressions levels of the genes GUCY1A1, PLS3, ARSD and CUX2 obtained from a prostate cancer sample of the subject, and determining the treatment response to ADT based on the GUCY1A1, PLS3, ARSD and CUX2 expression levels, wherein: a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds.

In a second aspect the invention relates to a computer program product comprising instructions which, when the program is executed by a computer, cause the computer to carry out a method for predicting a treatment response of a subject suffering from prostate cancer to androgen deprivation therapy (ADT) based on received expression levels for GUCY1A1, PLS3, ARSD and CUX2 determined in a prostate cancer sample of the subject, wherein the method comprises determining the treatment response based on the received expression levels, wherein a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds.

In a third aspect the invention relates to a kit of parts comprising primers and/or probes for specifically detecting and quantifying the expression level in biological sample of the genes GUCY1A1, PLS3, ARSD, and CUX2, preferably wherein the kit is a kit selected from an RNA sequencing kit, a PCR kit, a qPCR kit, a digital PCR kit, an immunohistochemistry kit, an ELISA kit, and an in situ RNA hybridization kit.

In a fourth aspect the invention relates to the use of the kit according to the third aspect of the invention in determining the treatment response of a subject suffering from prostate cancer to ADT, the use comprising using the primers and/or probes to quantify the expression levels of the genes GUCY1A1, PLS3, ARSD, and CUX2, and determining the treatment response based on the determined expression levels.

In a fifth aspect the invention relates to a non-steroidal androgen receptor inhibitor for use in the treatment, prevent or amelioration of prostate cancer in a subject as an androgen deprivation therapy, the use comprising: receiving values representing the expressions levels of the genes GUCY1A1, PLS3, ARSD and CUX2 obtained from a prostate cancer sample of the subject, and determining a treatment response to ADT based on the GUCY1A1, PLS3, ARSD and CUX2 expression levels, wherein: a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds, wherein the non-steroidal androgen receptor inhibitor is administered to the subject as a androgen deprivation therapy when a good treatment response is predicted.

### BRIEF DESCRIPTION OF THE DRAWINGS

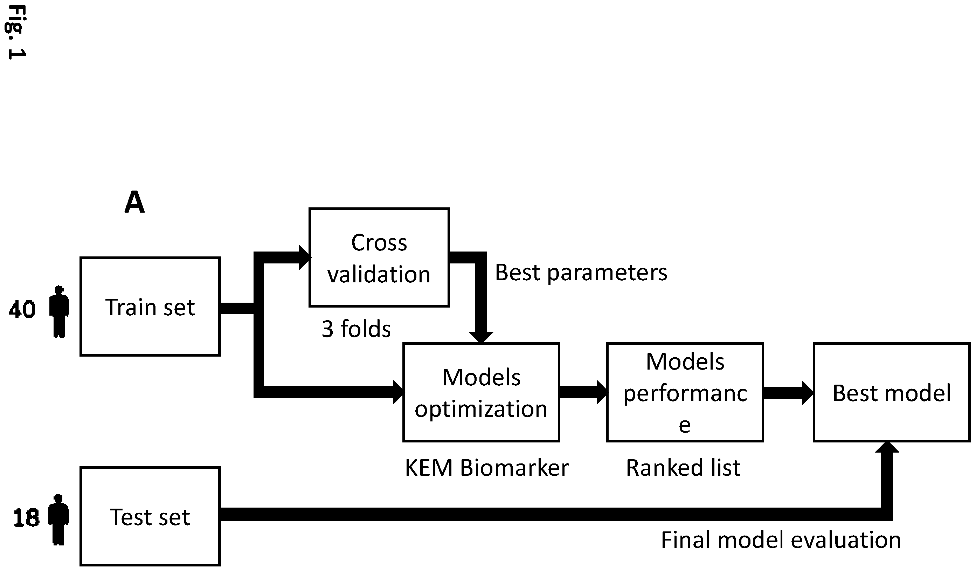

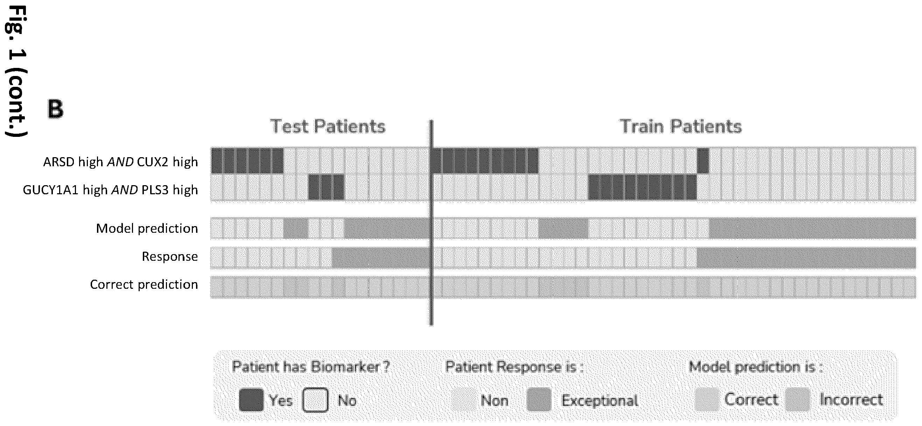

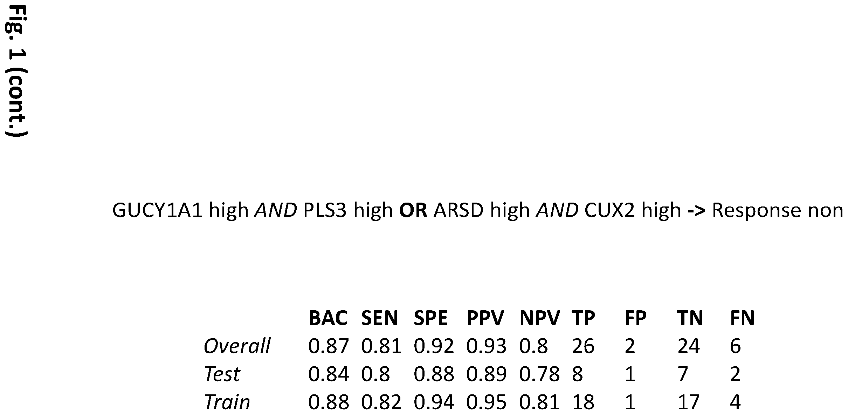

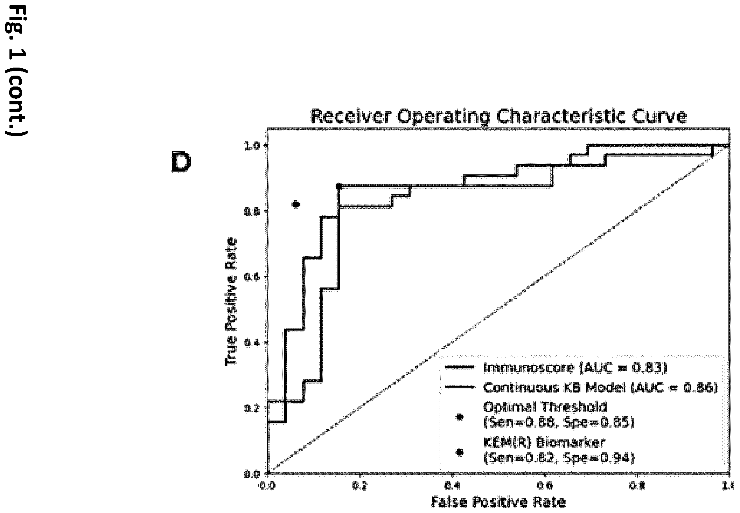

Fig. 1: Graphical abstract A. One run of the KEM^{®} biomarker pipeline. Multiple runs were computed in this analysis to evaluate the stability of the method for these data. B. Final model and coverage matrix: each patient is represented by a column, the model behavior and comparison with actual response is represented by the rows. C. performances of the final model and the immunoscore D. Receiver operating characteristic curve of the continuous model score and the immunoscore.

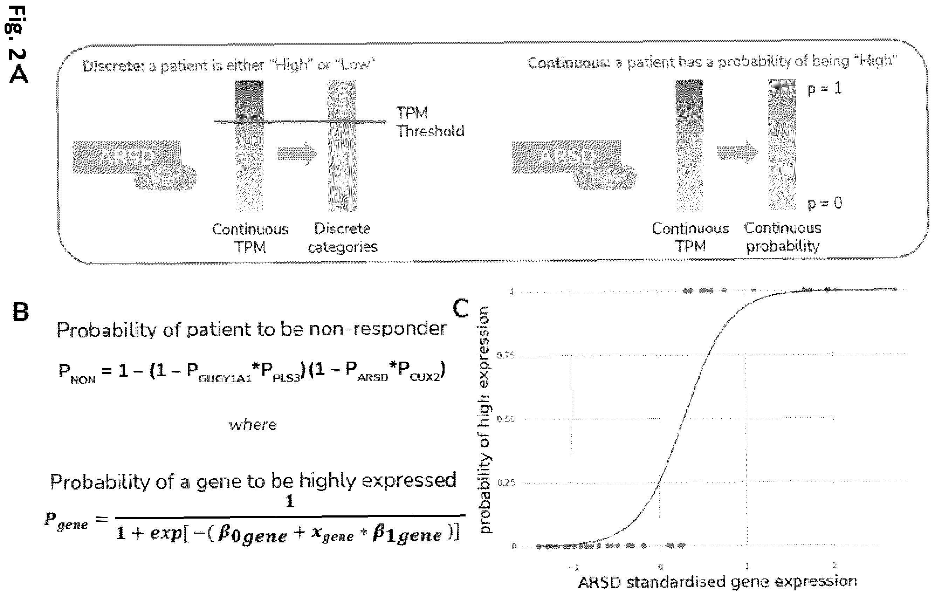

Fig. 2: Continuous score computation A. Standardized genes expression values are turned into a probability for each gene of the model. B. Formula of the continuous model score based on gene expression values for a given patient, with xa the gene expression of gene A, β0A and β1A the coefficients of the regression C. Logistic regression of a given gene of the model. A strong L2 regularization is applied to prevent overfitting.

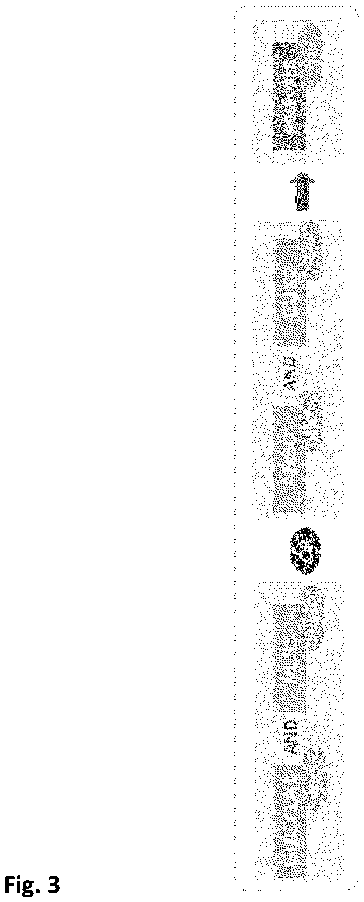

Fig. 3: Model composed of two rules, with two genes in each rule.

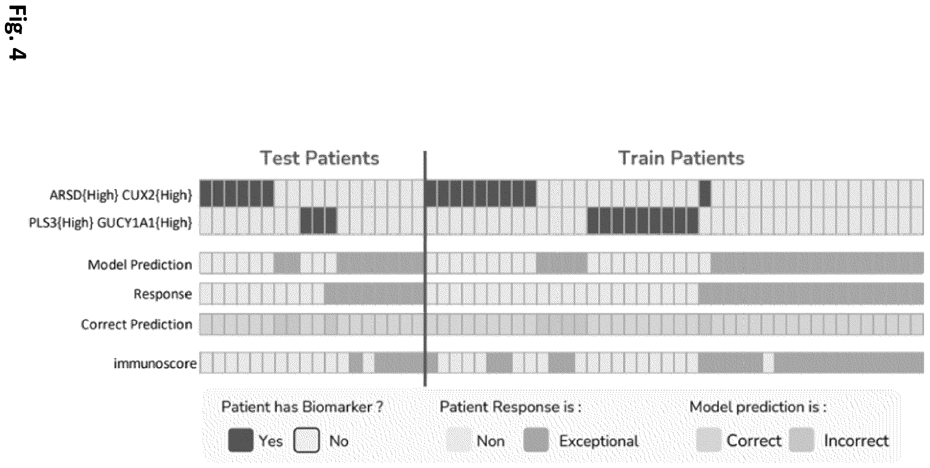

Fig. 4: Coverage matrix of the model: each patient is represented by a column. The first two rows represent the two rules inside the model, and whether they are verified or not for the patient. The *Model Prediction* can then be computed and compared with the actual *Response. Immunoscore* prediction using an optimal threshold is given as a reference.

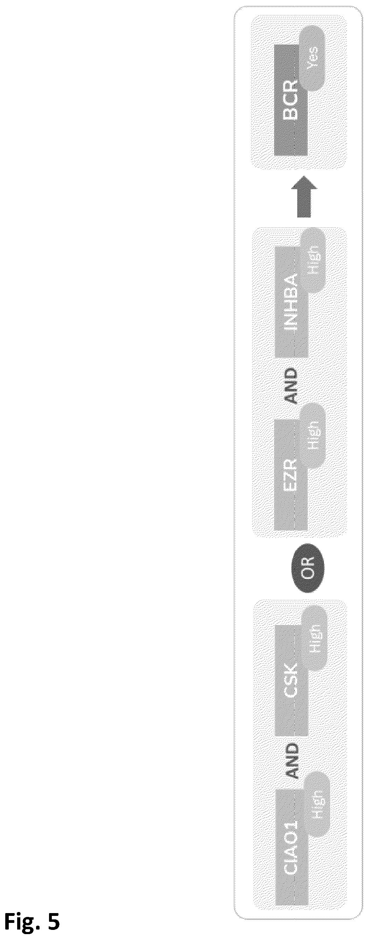

Fig. 5: Model composed of two rules, with two genes in each rule - based on the Darana method (Example 3).

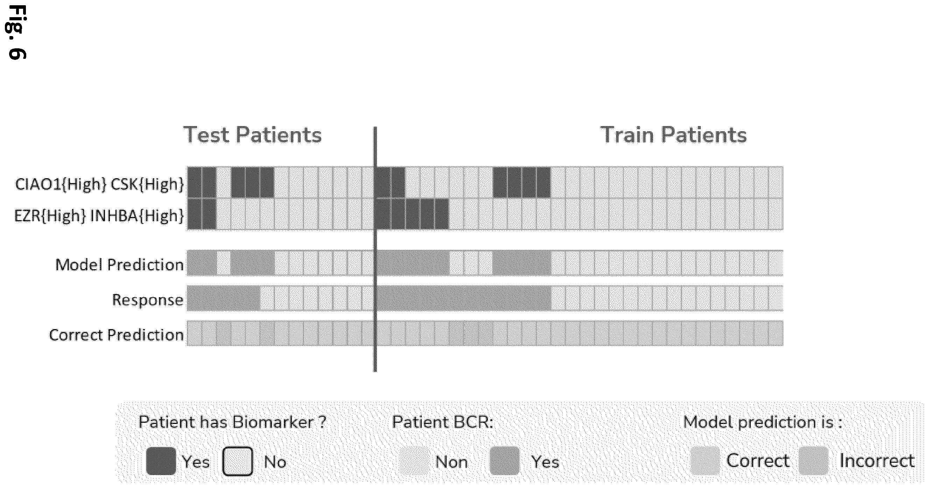

Fig. 6: Coverage matrix of the model: each patient is represented by a column. The first two rows represent the two rules inside the model, and whether they are verified or not for the patient. The *Model Prediction* can then be computed, and compared with the actual *Response.Immunoscore* prediction using an optimal threshold is given as a reference.

### Definitions

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20%, preferably ±15%, more preferably ±10%, and even more preferably ±5%.

"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

"Antagonist" and "inhibitor": These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

"Anti-cancer effect": This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

"Alleviating cancer": The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

"Compositions", "products" or "combinations": These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

"Combination therapy", or "in combination with": These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of" and "consisting of".

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject.

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

### DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention. Unless otherwise defined, all technical and scientific terms used herein have the same meaning as commonly understood by a person of ordinary skill in the art.

The present invention broadly relates to the finding that several AI derived models can be used to predict the outcome of ADT treatment of a prostate cancer patient. In an exemplary example such model is based on the expression levels of GUCY1A1, PLS3, ARSD and CUX2, but several other models can also be used and are described herein. It was found by the inventor that when either one of the following conditions was met:
- GUCY1A1 and PLS3 expression levels are high; and/or
- ARSD and CUX2 expression levels are high,
the developed model predicts a poor outcome of ADT treatment. When none of the above conditions are met (thus GUCY1A1 and PLS3 expression levels are both not high, and ARSD and CUX2 expression levels are both not high), a good outcome is predicted for ADT treatment. In addition, other predictive models were found and described below. The predictive model is useful as it can be used to provide a treatment recommendation for the patient. For example, when the model predicts a good outcome for ADT, it can be used as a confirmation to provide ADT, alternatively, when a poor outcome of ADT is predicted a different approach may be used, such as an alternative treatment or ADT supplemented with additional treatment.

Accordingly, in a first aspect the invention herein describes a computer implemented method for predicting a treatment response of a subject suffering from prostate cancer to androgen deprivation therapy (ADT), the method comprising: receiving values representing the expressions levels of the genes GUCY1A1, PLS3, ARSD and CUX2 obtained from a prostate cancer sample of the subject, and determining the treatment response to ADT based on the GUCY1A1, PLS3, ARSD and CUX2 expression levels, wherein: a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds.

The method described herein may further include an active step of determining expression levels, therefore in an embodiment the invention relates to a method for predicting a treatment response of a subject suffering from prostate cancer to androgen deprivation therapy (ADT), the method comprising: determining the expressions levels of the genes GUCY1A1, PLS3, ARSD and CUX2 in a prostate cancer sample obtained from the subject, and determining the treatment response to ADT based on the GUCY1A1, PLS3, ARSD and CUX2 expression levels, wherein: a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds.

When used herein the term subject refers to a human or a non-human animal such as a mammal. Preferably the subject is a human subject. The terms subject and patient are used interchangeably herein. The subject is a subject diagnosed with prostate cancer. Preferably the subject has localized prostate cancer. The methods as broadly described herein is preferably performed prior to starting treatment.

Androgen deprivation therapy also called androgen ablation therapy or androgen suppression therapy, is an antihormone therapy whose main use is in treating prostate cancer. ADT reduces the levels of androgen hormones, with drugs or surgery, to prevent the prostate cancer cells from growing. Surgical based ADT includes orchiectomy (surgical castration by removing of the testicles). Drug based ADT can be chemical castration or antiandrogen therapy. Chemical castration can be performed by LHRH agonists and antagonists, which both lower the amount of testosterone made by the testicles. Antiandrogen therapy can use steroidal or non-steroidal antiandrogens. Thus when referred herein to ADT the therapy may be one or more selected from orchiectomy, chemical castration and antiandrogen therapy. Preferably the ADT refers to antiandrogen therapy, more preferably to non-steroidal antiandrogen therapy.

Non limiting examples of LHRH agonists, also referred to as GnRH agonists, are Azagly-nafarelin, Buserelin, Deslorelin, Fertirelin, Gonadorelin, Goserelin, Histrelin, Lecirelin, Leuprorelin, Nafarelin, Peforelin, and Triptorelin. Thus in an embodiment the LHRH agonist (GnRH agonist) is selected from Azagly-nafarelin, Buserelin, Deslorelin, Fertirelin, Gonadorelin, Goserelin, Histrelin, Lecirelin, Leuprorelin, Nafarelin, Peforelin, and Triptorelin.

Non limiting examples of steroidal antiandrogens are Abiraterone acetate, Allylestrenol, Chlormadinone acetate, Cyproterone acetate, Delmadinone acetate, Gestonorone caproate, Hydroxyprogesterone caproate, Medroxyprogesterone acetate, Megestrol acetate, Osaterone acetate, Oxendolone, and Spironolactone. Thus in an embodiment the steroidal antiandrogen is selected from Abiraterone acetate, Allylestrenol, Chlormadinone acetate, Cyproterone acetate, Delmadinone acetate, Gestonorone caproate, Hydroxyprogesterone caproate, Medroxyprogesterone acetate, Megestrol acetate, Osaterone acetate, Oxendolone, and Spironolactone.

Non limiting examples of non-steroidal androgen receptor inhibitor are Flutamide, Nilutamide, Bicalutamide, Enzalutamide, Clascoterone, Apalutamide, Proxalutamide, Cimetidine, Topilutamide, and Darolutamide. Thus in an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide.

Androgen deprivation therapy may be provided as adjuvant androgen deprivation therapy, neoadjuvant androgen deprivation therapy, or salvage androgen deprivation therapy, among others. Thus in an embodiment the ADT is provided as adjuvant androgen deprivation therapy, neoadjuvant androgen deprivation therapy, or salvage androgen deprivation therapy. In a particularly preferred embodiment the ADT is neoadjuvant androgen deprivation therapy or adjuvant androgen deprivation therapy, more preferably neoadjuvant androgen deprivation therapy.

The term adjuvant androgen deprivation therapy (AADT) when used herein refers to the provision of androgen deprivation therapy after surgery (e.g. partial or complete removal of the prostate) or radiotherapy.

Neoadjuvant androgen deprivation therapy (NADT) is a recommended treatment for patients diagnosed with localized prostate cancer. NADT is systemic therapy administered after the diagnosis of prostate cancer but before locoregional therapy such as radical prostatectomy (RP) or radiation. With NADT prior to RP, the intent is to eradicate malignant androgen-dependent cells, in the hope that sufficient tumor regression will permit complete resection of residual prostate cancer, improving pathologic outcome and survival. The role of preoperative androgen deprivation remains controversial, however. The present methods, products, and uses aim to identify those patients for whom NADT likely is beneficial, and to identify those patients whom are more likely to not benefit form NADT and thus should receive an alternative or additional treatment.

The term salvage androgen deprivation therapy (SADT) when used herein refers to the use of ADT to treat biochemical recurrence, metastasis or tumor growth after treatment of prostate cancer, typically by surgery or radiation. Biochemical recurrence (BCR) indicates a rise of prostate-specific antigen (PSA) levels in the blood of a prostate cancer patient after treatment with surgery or radiation. Typically SADT is performed well after (e.g. one year or more) after initial treatment of the prostate cancer by for example surgery or radiation. Thus SADT is used to treat relapse of prostate cancer.

Accordingly, the methods as broadly described herein are preferably performed prior to initiation of treatment, or in the case of SADT before start of secondary treatment, for example shortly after diagnosis of prostate cancer or after confirmation of BCR. For example, after confirmation of prostate cancer by biopsy, the biopsy may be used as a prostate cancer sample to predict a treatment response to ADT, e.g. AADT, NADT, or SADT. The predicted response to ADT may be used to suggest or determine a treatment strategy. For example if a good response is predicted to ADT, the recommended treatment is ADT (e.g. AADT, NADT or SADT), and if a poor response is predicted the recommended treatment is ADT supplemented with additional therapy or an alternative treatment to ADT. Thus in an embodiment the method is performed before start of treatment or secondary treatment. In an embodiment the prostate cancer sample is obtained before start of treatment. In an embodiment the treatment is decided or suggested based on the treatment outcome predicted by the method of the invention.

Herein, the term "outcome" relates to a specific result or effect that can be measured. Examples of an outcome of a subject having prostate cancer include an outcome of the prostate cancer, a pathology outcome, an outcome of a therapy for treating prostate cancer, such as a ADT outcome or more specifically AADT, NADT or SADT outcome, as well as other outcomes, such as a biomarker related - e.g. biochemical recurrence, a genomic profile related outcome, an imaging related outcome (e.g., a change in morphology of the tumor), a biology related outcome (e.g., metastasis or recurrence of the tumor), a surrogate marker related outcome, a quality of life outcome, a cancer specific survival outcome, and an overall survival outcome.

The inventors used AI to identify expression level based models to predict an outcome for ADT. To do this gene expression levels based models were trained on a training cohort and tested on a test cohort. One of the best scoring models is described here in detail, but other models also achieve good results and are also described herein.

The model uses the expression levels of the genes GUCY1A1, PLS3, ARSD and CUX2. For each of these expression levels it is determined whether the expression level is high or not high. This may for example be done by setting a threshold expression level, above which the expression level is defined as high, and below which the expression level is defined as not high or low. Such threshold can easily be determined in a reference sample, or preferably a cohort of reference samples. For this the expression level is preferably quantified. Quantification may for example be done by normalizing to a household gene or by calculating reads per million, but other suitable method for quantifying gene expression levels are known in the art and may also be suitably used in the method described herein. Thus when used herein the term "high expression level" or "the expression level is high" can be interpreted as exceeding a threshold expression level for said gene. In this respect it is appreciated that a high expression level will be different for each gene and thus for each gene individually it needs to be determined what constitutes a high expression level, for example by establishing the appropriate threshold above which it is deemed high. The threshold may be determined using methods common in the art. For example from a pool of prostate cancer patients which includes responders and non-responders to ADT expression levels may be collected for the respective gene and the threshold may be set as the mean or average expression level of this pool. Alternatively average expression levels may be determined for responders and non-responder and the threshold may be set as a value in between those average expression level values. The threshold is preferably chosen such as to optimize separation between the responder and non-responder groups.

Gene expression levels may be determined using techniques common in the art such a but not limited to RNA sequencing, targeted sequencing, PCR, digital PCR, and quantitative PCR. Gene expression levels may be determined using means for determining expression levels of the respective genes. The means for determining the expression levels of the genes described herein may be primers and/or probes suitable for any one of PCR, quantitative PCR, digital PCR, RNA sequencing, or targeted RNA sequencing. Thus for example the means may be PCR primers, quantitative PCR primers and probes, digital PCR primers and probes, or capture probes for targeted mRNA sequencing. Preferably the means are PCR or qPCR amplification primers and optionally probes or mRNA targeted sequencing capture probes.

Thus when used herein a "value representing an expression level" refers to the quantification of said expression level reflected in a value. Such value may for example be expressed as transcripts per million or a normalized expression level. Expression levels may be normalized using one or more household genes. In one realization, the gene expression level for each of GUCY1A1, PLS3, ARSD, and CUX2 is normalized with respect to one or more household genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these household genes.

Thus in an embodiment the expression levels thresholds for each of GUCY1A1, PLS3, ARSD, and CUX2 are predetermined in a cohort of prostate cancer samples from patients having received ADT.

In the present finding it is described that a poor outcome is predicted in case that at least one of the conditions is met that (1) GUCY1A1 and PLS3 expression levels are high; and/or
(2) ARSD and CUX2 expression levels are high. If neither of this condition is met a good outcome is predicted. For example: if GUCY1A1 and PLS3 expression levels are high and ARSD and CUX2 expression levels are low, a poor outcome is predicted because the first criteria is met. If GUCY1A1 and PLS3 expression levels are high and ARSD and CUX2 expression levels are high, a poor outcome is predicted because both the first and the second criteria are met. If GUCY1A1 and PLS3 expression levels are low and ARSD and CUX2 expression levels are high, a poor outcome is predicted because the second criteria is met. If GUCY1A1 and PLS3 expression levels are low and ARSD and CUX2 expression levels are low, a good outcome is predicted because none of the criteria are met. If GUCY1A1 expression levels are high and PLS3, ARSD and CUX2 expression levels are low, a good outcome is predicted because none of the criteria are met. If GUCY1A1 and CUX2 expression levels are high and PLS3, and ARSD expression levels are low, a good outcome is predicted because none of the criteria are met.

The term "GUCY1A1" refers to the human Guanylate Cyclase 1 Soluble Subunit Alpha 1 gene (Ensembl: ENSG00000164116), for example, to the sequence as defined in NCBI Reference Sequence NM_000856.6, specifically, to the nucleotide sequence as set forth in SEQ ID NO: 1, which correspond to the sequences of the above indicated NCBI Reference Sequences of the GUCY1A1 transcript, and also relates to the corresponding amino acid sequence for example as set forth in SEQ ID NO:2, which correspond to the protein sequences defined in NCBI Protein Accession Reference Sequence NP_000847.2 encoding the GUCY1A1 polypeptide.

The term "GUCY1A1" also comprises nucleotide sequences showing a high degree of homology to GUCY1A1, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1.

The term "PLS3" refers to the human Plastin 3 gene (Ensembl: ENSG00000102024), for example, to the sequence as defined in NCBI Reference Sequence NM_001136025.5, specifically, to the nucleotide sequence as set forth in SEQ ID NO:3, which correspond to the sequences of the above indicated NCBI Reference Sequences of the PLS3 transcript, and also relates to the corresponding amino acid sequence for example as set forth in SEQ ID NO:4, which correspond to the protein sequences defined in NCBI Protein Accession Reference Sequence NP_001129497.1 encoding the PLS3 polypeptide.

The term "PLS3" also comprises nucleotide sequences showing a high degree of homology to PLS3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or amino acid sequences being at least 75%, 80%, 85%, 90%, 91%, 92%, 93%, 94%, 95%, 96%, 97%, 98% or 99% identical to the sequence as set forth in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3.

The term "ARSD" refers to the human Arylsulfatase D gene (Ensembl: ENSG00000006756), for example, to the sequence as defined in NCBI Reference Sequence NM_001669.4, specifically, to the nucleotide sequence as set forth in SEQ ID NO:5, which correspond to the sequences of the above indicated NCBI Reference Sequences of the ARSD transcript, and also relates to the corresponding amino acid sequence for example as set forth in SEQ ID NO:6, which correspond to the protein sequences defined in NCBI Protein Accession Reference Sequence NP_001660.2 encoding the ARSD polypeptide.

The term "ARSD" also comprises nucleotide sequences showing a high degree of homology to ARSD, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "CUX2" refers to the human Cut Like Homeobox 2 gene (Ensembl: ENSG00000111249), for example, to the sequence as defined in NCBI Reference Sequence NM_001370598.1, specifically, to the nucleotide sequence as set forth in SEQ ID NO:7, which correspond to the sequences of the above indicated NCBI Reference Sequences of the CUX2 transcript, and also relates to the corresponding amino acid sequence for example as set forth in SEQ ID NO:8, which correspond to the protein sequences defined in NCBI Protein Accession Reference Sequence NP_001357527.1 encoding the CUX2 polypeptide.

The term "CUX2" also comprises nucleotide sequences showing a high degree of homology to CUX2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The expression levels are determined in a prostate cancer sample obtained from the subject. When used herein the term prostate cancer sample refers to a sample comprising at least one prostate cancer cell. The sample may for example be a tumor biopsy, part of a resected prostate, a tissue resection, or a blood sample comprising circulating tumor cells. In addition, the prostate cancer sample may include body tissue and/or a fluid, such as, but not limited to, blood (or blood derived such as serum, plasma, or PBMC's (peripheral blood mononuclear cells)), sweat, saliva, urine, bone marrow, and a needle biopsy or resection biopsy, preferably comprising at least one prostate cancer cell. Furthermore, the biological sample may contain a cell extract derived from or a cell population including a cancerous cell or a cell derived from tissue suspected to be cancerous, such as a myeloid cell. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumor cells, extracellular vesicles, a sample containing prostate secreted exosomes, or cell lines or cancer cell line.

In one particular realization, a prostate cancer sample such as a biopsy or resection sample may be obtained and/or used. Such samples may include prostate cancer cells or cell lysates.

It is also conceivable that the content of a prostate cancer sample is submitted to an enrichment step. For instance, a sample may be contacted with ligands specific for the cell membrane or organelles of certain cell types, e.g., tumor cells or myeloid cells, functionalized for example with magnetic particles. The material concentrated by the magnetic particles may subsequently be used for detection and analysis steps as described herein above or below.

Furthermore, prostate cancer cells, e.g., tumor cells, may be enriched via filtration processes of fluid or liquid samples, e.g., blood, urine, etc. Such filtration processes may also be combined with enrichment steps based on ligand specific interactions as described herein above.

When used herein the term prostate cancer cell refers to a tumor cell derived from the prostate or a metastasis thereof. It is preferred that a prostate cancer sample as provided herein is obtained from the subject before the start of the therapy. It is also preferred that said biological sample is a bladder sample or a bladder cancer sample. Thus, in a preferred embodiment the method according to the invention comprises that the prostate cancer sample is obtained from the subject before the start of the therapy, preferably wherein the prostate cancer sample is a prostate sample or a sample comprising prostate cancer cells from the subject. Alternatively, the method according to the invention comprises providing the prostate cancer sample obtained from the subject before the start of the therapy, preferably wherein the prostate cancer sample is a prostate sample or a sample comprising prostate cancer cells from the subject.

When used herein a good outcome preferably indicates an exceptional response. When used herein, the term exceptional response indicates pathologic complete response or minimal residual disease. The method may thus predict an increased or high chance of having an exceptional response when the expression levels of GUCY1A1 and PLS3 do both not exceed predetermined expression level thresholds, and the expression levels of ARSD and CUX2 do both not exceed predetermined expression level thresholds. A good outcome may also be a reduced chance of a non-favorable outcome occurring. Non-limiting examples of non-favorable outcomes are: biochemical recurrence, metastasis, cancer specific death or prostate cancer specific death.

When used herein a poor outcome preferably indicates a response that is not an exceptional response. For example, a poor response may be a partial response or no response. The method may thus predict an increased or high chance of having a partial response or no response when the expression levels of both GUCY1A1 and PLS3 exceed predetermined expression level thresholds, and/or when expression levels of both ARSD and CUX2 exceed predetermined expression level thresholds. A poor outcome may also be an increased chance of a non-favorable outcome occurring. Non-limiting examples of non-favorable outcomes are: biochemical recurrence, metastasis, cancer specific death or prostate cancer specific death.

The methods as broadly described herein predict the outcome of androgen deprivation therapy, e.g. AADT, NADT or SADT. In an embodiment the androgen deprivation therapy comprises treatment with a non-steroidal androgen receptor inhibitor. In an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide. In an embodiment the non-steroidal androgen receptor inhibitor. In an embodiment the non-steroidal androgen receptor inhibitor is Enzalutamide. In an embodiment the androgen deprivation therapy comprises treatment with a non-steroidal androgen receptor inhibitor combined with a different treatment. In an embodiment the androgen deprivation therapy further comprises treatment with one or more of: a CYP17A1 inhibitor, an antigonadotropin, and an glucocorticoid receptor agonist. Non limiting examples of CYP17A1 inhibitors are Abiraterone acetate, Ketoconazole, and Seviteronel. Preferably the CYP17A inhibitor is Abiraterone acetate. Non limiting examples antigonadotropins are Estrogens such as estradiol and its esters, ethinylestradiol, conjugated estrogens, diethylstilbestrol, GnRH analogues such as GnRH agonists (e.g., goserelin, leuprorelin) or GmRH antagonists (e.g., cetrorelix) or Progestogens (e.g., chlormadinone acetate, cyproterone acetate, gestonorone caproate, medroxyprogesterone acetate, megestrol acetate). Preferably the antigonadotropin is a GnRH analogue, more preferably a GnRH agonist, most preferably leuprorelin. Non limiting examples of glucocorticoid receptor agonists are prednisone, Dagrocorat, Fosdagrocorat, and Mapracorat. In a preferred embodiment the glucocorticoid receptor agonist is prednisone. Thus in an embodiment the androgen deprivation therapy further comprises treatment with one or more, for example one, two or all, of Leuprolide, Abiraterone Acetate, and Prednisone.

In an embodiment the method further comprises a step of providing the prediction of the treatment response to the subject or a medical caregiver. In an embodiment the method further comprises a step of providing a recommended treatment strategy to the subject or a medical caregiver. In an embodiment the recommended treatment strategy comprises recommending ADT when a good treatment response is predicted to treatment with ADT. In an embodiment the recommended treatment strategy comprises recommending an alternative treatment or ADT supplemented with additional treatment when a poor treatment response is predicted to treatment with ADT. In an embodiment the alternative treatment consist or comprises of chemotherapy, immunotherapy, targeted therapy or PDE4D7 expression or activity increasing therapy. In an embodiment the additional treatment consist or comprises of chemotherapy, immunotherapy, targeted therapy or PDE4D7 expression or activity increasing therapy.

When used herein, a gene therapy for inducing PDE4D7 expression refers to a method to introduce a nucleotide encoding the phosphodiesterase 4D7 isoform in a cell, preferably a cell of a subject having prostate cancer, more preferably in a prostate cancer cell or a metastasis thereof. The nucleotide may for example be a viral vector or a non-viral vector, however it is understood that any nucleotide which can be introduced in a cell of a patient and express PDE4D7 may be used. For example the nucleotide may also be an mRNA encoding PDE4D7 or a variant of PDE4D7. Nucleotides such as mRNA may be expressed in a tumor cell in vivo using lipid nanoparticles (LNP). Suitable LNP formulations are known to the skilled person.

Viral vectors are a known strategy for gene therapy, typically viral-vector gene therapies use modified viruses as drug-delivery vehicles to introduce specific DNA or RNA sequences into cells. The vector is packaged using the viral proteins allowing infection of cells and expression of its viral genome. Typically, the viral vector is modified such that no new viral particles can be produced upon infection of the target cell. Non limiting examples include retroviruses (RV), adenoviruses (AV), adeno-associated viruses (AAV), lentiviruses (LV), and herpes simplex viruses (HSV). Other ways to use viral particles or viral vectors to introduce the PDE4D7 encoding nucleotide are known to the person skilled in the art and also envisaged to be encompassed by the invention.

Alternatively, to viral vector based gene therapy, a non-viral vector may be used. These vectors typically contain a promotor to drive expression of the relevant construct (e.g. PDE4D7), and typically require some means to introduce the vector into the target cell. Means to deliver a non-viral vector to a target cell in a subject are known to the skilled person, and for example reviewed in Ramamoorth M, Narvekar A. Non-viral vectors in gene therapy- an overview. J Clin Diagn Res. 2015 Jan;9(1):GE01-6 (hereby incorporated by reference in its entirety). For example, nanoparticles can be used to encapsulate the vector. Non limiting examples are lipid based nanoparticles, peptide based nanoparticles, cationic lipid based nanoparticles, apolipoprotein based nanoparticles, (synthetic) polymer based nanoparticles such as polyethylenimine (PEI), chitosan, polylactate, polylactide, polyglucoside, denriomer or polymethracylate based nanoparticles. When used herein a nanoparticle is a small particle which can be used a sa carrier to deliver a cargo (payload) in a patient. Preferably the cargo is the product as broadly defined herein. Therefore a nanoparticle can be used to deliver the product induces the expression of PDE4D7 or promotes activity of phosphodiesterase 4D7 to a site in patient, e.g. a cell, tissue or organ. Other ways to use non- viral vectors to introduce the PDE4D7 encoding nucleotide are known to the person skilled in the art and also envisaged to be encompassed by the invention.

Therefore, in an embodiment the gene therapy is selected from: a viral vector capable of expressing PDE4D7 in a subject or a non-viral vector capable of expressing PDE4D7 in a subject. In an embodiment the product is delivered using a nanoparticle.

When used herein the term vector is used to indicate any particle (e.g., a plasmid, a cosmid, a Lambda phage) used as a vehicle to artificially carry a foreign nucleic sequence - usually DNA - into another cell, where it can be replicated and/or expressed.

When used herein a compound that induces PDE4D7 expression is intended to refer to any biological or chemical compound that is able to increase the expression of PDE4D7. This may for example be achieved by promoting transcription of the PDE4D7 gene or inhibiting degradation of the phosphodiesterase 4D7 isoform protein. For example the compound may engage a signal transduction pathway to indirectly promote transcription of the PDE4D7 or directly interact with the promoter region of the genomic DNA to promote transcription of the PDE4D7 isoform.

When used herein promoting transcription of PDE4D7 refers to increasing the transcription of PDE4D7 resulting in a higher amount of PDE4D7 mRNA transcripts, and/or preferably in a higher amount of phosphodiesterase 4D7 isoform protein in the cell. The promoting may be specific for PDE4D7, specific for all PDE4D isoforms, specific for all PDE4 or even all PDE family members. In other words, increasing expression of PDE4D7 does not exclude that other PDE4D isoforms, other PDE4 family members or even other PDE family members are also increased in expression.

When used herein, a compound directly stimulating or promoting phosphodiesterase 4D7 activity is intended to refer to a compound which directly engages with the phosphodiesterase 4D7 isoform protein enzymatic activity. Without wishing to be bound be theory, it is theorized that PDE proteins such as the PDE4D7 protein can exist in an active and inactive conformation, wherein activity can be induced by other compounds through interaction with the PDE protein, for example by promoting an active conformation of the protein or by blocking or preventing binding of inhibitors, or by modifying the protein to promote activity (for example by phosphorylation or other known protein modifications). When used herein, enzymatic activity when referring to a phosphodiesterase refers to catalysis of the hydrolysis of cAMP and/or cGMP.

When used herein, a compound indirectly stimulating or promoting phosphodiesterase 4D7 activity is intended to refer to a compound which indirectly engages with the phosphodiesterase 4D7 isoform protein enzymatic activity. Similarly as above it is envisaged that compounds may induce activators of PDE4D7 or block inhibitors form PDE4D7 activity and thus indirectly affect the protein's enzymatic activity. The person skilled in the art is aware of methods to determine PDE or specifically PDE4D7 activity. For example, commercial products are available to assay for PDE activity, and methods have for example been described in Blair et al. Measuring cAMP Specific Phosphodiesterase Activity: A Two-step Radioassay. Bio Protoc. 2020 Apr 5;10(7):e3581, hereby incorporated in its entirety by reference.

When used herein, an inhibitor of a transcriptional inhibitor of PDE4D7 refers to a compound capable of blocking an inhibitor of PDE4D7 gene transcription. The inhibitor of PDE4D7 gene transcription may be a direct inhibitor capable of binding the genomic DNA and preventing or reducing gene transcription or an indirect inhibitor which through downstream actions reduces or inhibits transcription of the PDE4D7 gene.

When used herein an inhibitor of an inhibitor of phosphodiesterase 4D7 enzymatic activity refers to a compound that can block an inhibitor of phosphodiesterase isoform 4D7 protein activity. For example the compound may function by degrading the inhibitor, preventing the inhibitor from engaging with PDE4D7 or by inhibiting the activity of the inhibitor.

When used herein, mRNA encoding a phosphodiesterase 4D7 protein refers to a RNA molecule from which the protein corresponding to SEQ ID NO: 20 or a substantially similar protein can be translated. The mRNA molecule generally comprises non translated elements such as 5' and 3' UTRs. It is anticipated that by direct introduction of PDE4D7 mRNA into the target cell (e.g. a prostate cancer cell in a subject) PDE4D7 can by upregulated. Method of delivering mRNA to a target cell are known to the skilled person, for example using nanobodies as described above.

When used herein a phosphodiesterase 4D7 protein refers to a compound comprising PDE4D7 protein or a biosimilar, or a constitutively active variant thereof, having phosphodiesterase activity. It is anticipated that by direct introduction of PDE4D7 protein into the target cell (e.g. a prostate cancer cell in a subject) PDE4D7 activity can be increased. Method of delivering protein to a target cell are known to the skilled person, for example using nanobodies as described above.

When used herein, a phosphodiesterase 4D7 activity promoting peptide refers to a peptide that is able to increase activity of the PDE4D7 enzyme. Non limiting examples are Amyloid beta (Abeta) peptides, which have been demonstrated to specifically increase long PDE4D isoforms such as PDE4D7. When used herein Amyloid beta, also known as Aβ or Abeta, refers to peptides of 37-49 amino acids (Chen et al. Acta Pharmacol Sin 38, 1205-1235 (2017)) that are the main component of the amyloid plaques found in the brains of people with Alzheimer's disease. Amyloid beta peptide (Aβ) is produced through the proteolytic processing of a transmembrane protein, amyloid precursor protein (APP), by β- and γ-secretases.

When used herein the term chemotherapy refers to a chemotherapeutic agents, also known as cytotoxic agents or cytostatic drugs, that are known to be of use in chemotherapy for cancer, and have the intention to kill tumor cells or reduce tumor size. Non limiting examples are Alkylating agents such as Altretamine, Bendamustine, Busulfan, Carboquone, Carmustine, Chlorambucil, Chlormethine, Chlorozotocin, Cyclophosphamide, Dacarbazine, Fotemustine, Ifosfamide, Lomustine, Melphalan, Melphalan flufenamide, Mitobronitol, Nimustine, Nitrosoureas, Pipobroman, Ranimustine, Semustine, Streptozotocin, Temozolomide, Thiotepa, Treosulfan, Triaziquone, Triethylenemelamine, Trofosfamide, and Uramustine; or Anthracyclines such as Aclarubicin, Daunorubicin, Doxorubicin, Epirubicin, Idarubicin, Mitoxantrone, Pirarubicin, Valrubicin, and Zorubicin; or Cytoskeletal disruptors (taxanes) such as Abraxane, Cabazitaxel, Docetaxel, Larotaxel, Paclitaxel, Taxotere, and Tesetaxel; or Epothilones such as Ixabepilone; or Histone deacetylase inhibitors such as Entinostat, Romidepsin, Vorinostat, and Zabadinostat; or Inhibitors of topoisomerase I such as Belotecan, Camptothecin, Exatecan, Gimatecan, Irinotecan, and Topotecan; or Inhibitors of topoisomerase II such as Etoposide, Teniposide, and Tafluposide; or Kinase inhibitors such as Bortezomib, Erlotinib, Gefitinib, Imatinib, Vemurafenib, and Vismodegib; or Nucleotide analogs and precursor analogs such as Azacitidine, Azathioprine, Capecitabine, Cladribine, Clofarabine, Cytarabine, Decitabine, Doxifluridine, Fludarabine, Fluorouracil, Gemcitabine, Hydroxyurea, Mercaptopurine, Methotrexate, Nelarabine, Pemetrexed, and Tioguanine (formerly Thioguanine); or Peptide antibiotics such as Actinomycin, and Bleomycin; or Platinum-based agents such as Main Carboplatin, Cisplatin, Dicycloplatin, Oxaliplatin, Nedaplatin, and Satraplatin; or Retinoids such as Alitretinoin, Bexarotene, and Tretinoin; or Vinca alkaloids and derivatives such as Vinblastine, Vincristine, Vindesine, and Vinorelbine.

When used herein targeted therapy may refer to targeted radionuclide therapy. Targeted radionuclide therapy refers to radioisotopes bound to or incorporated in a targeting agent such as PSMA or an antibody such as a nanobody. A non-limiting example suitable for prostate cancer is PSMA Lutetium-177. Targeted therapy may also refer to a therapy that acts on a protein or pathway particularly re;lied on by cancer cells. For example a targeted therapy for prostate cancer may target PARP (PARP inhibitors). Non limiting examples of PARP inhibitors are Rucaparib, Olaparib, Talazoparib, and Niraparib plus abiraterone.

Non limiting examples of immunotherapy are cancer vaccine (such as but not limited to Sipuleucel-T (Provenge) and prostatic acid phosphatase (PAP)), or Immune checkpoint inhibitors, such as but not limited to Ipilimumab, Nivolumab, Pembrolizumab, Cemiplimab, Spartalizumab, Camrelizumab, Sintilimab, Tislelizumab, Toripalimab, Dostarlimab, Retifanlimab, AMP-224, MED10680, Atezolizumab, Avelumab, Durvalumab, Envafolimab, Cosibelimab, AUNP-12, CA-170, anti-OX40 antibody BMS 986178, Tremelimumab, Abatacept, Belatacept, Sotorasib, Adagrasib, Relatlimab, Lisavanbulin, PHI-101, and Quemliclustat.

In a second aspect the invention relates to a computer program product comprising instructions which, when the program is executed by a computer, cause the computer to carry out a method for predicting a treatment response of a subject suffering from prostate cancer to androgen deprivation therapy (ADT) based on received expression levels for GUCY1A1, PLS3, ARSD and CUX2 determined in a prostate cancer sample of the subject, wherein the method comprises determining the treatment response based on the received expression levels, wherein a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds. In an embodiment the ADT is AADT, NADT or SADT, preferably AADT or NADT, more preferably NADT.

Further disclosed herein is a method of treating, preventing or ameliorating a subject with prostate cancer, the method comprising: predicting a treatment response of a subject suffering from prostate cancer to androgen deprivation therapy (ADT) by receiving values representing the expressions levels of the genes GUCY1A1, PLS3, ARSD and CUX2 obtained from a prostate cancer sample of the subject, and determining the treatment response to ADT based on the GUCY1A1, PLS3, ARSD and CUX2 expression levels, wherein: a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds; and treating the subject by ADT when a good outcome to ADT is predicted and administering to the subject ADT supplemented with chemotherapy, immunotherapy, targeted therapy or PDE4D7 expression or activity increasing therapy or chemotherapy, immunotherapy, targeted therapy or PDE4D7 expression or activity increasing therapy without ADT when a poor outcome to ADT is predicted. In an embodiment the ADT is AADT, NADT or SADT, preferably AADT or NADT, more preferably NADT.

In an embodiment the ADT, e.g. AADT, NADT or SADT, consists or comprises of administering non-steroidal androgen receptor inhibitor to the subject prior to radical prostatectomy or radiation therapy. In an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide. In an embodiment the ADT consists or comprises of administering non-steroidal androgen receptor inhibitor combined with one or more of: a CYP17A1 inhibitor, an antigonadotropin, and an glucocorticoid receptor agonist to the subject.

In a third aspect the invention relates to a kit of parts comprising primers and/or probes for specifically detecting and quantifying the expression level in biological sample of the genes GUCY1A1, PLS3, ARSD, and CUX2, preferably wherein the kit is a kit selected from an RNA sequencing kit, a PCR kit, a qPCR kit, a digital PCR kit, an immunohistochemistry kit, an ELISA kit, and an in situ RNA hybridization kit. In an embodiment the kit is for selectively amplifying GUCY1A1, PLS3, ARSD, and CUX2 mRNA in a sample. In an embodiment the kit is for detecting GUCY1A1, PLS3, ARSD, and CUX2 protein in a sample. In an embodiment the kit is for quantifying GUCY1A1, PLS3, ARSD, and CUX2 mRNA or protein. In an embodiment the kit is suitable for use in a method for determining a treatment response to ADT, e.g. AADT, NADT, or SADT.

The kit may further comprise buffers, components or enzymes suitable for the envisioned technique, e.g. RNA sequencing, PCR, qPCR, digital PCR, immunohistochemistry, ELISA, or in situ RNA hybridization. Thus the kit may comprise one or more of a polymerase, a buffer suitable for PCR reactions, dNTP stock solutions, a buffer suitable for RNA sequencing, a primary and/or second antibody for ELISA, a reagent or colorant for detecting ELISA signal, and in situ hybridization buffer.

In a fourth aspect the invention relates to the use of the kit as broadly described herein in determining the treatment response of a subject suffering from prostate cancer to ADT, the use comprising using the primers and/or probes to quantify the expression levels of the genes GUCY1A1, PLS3, ARSD, and CUX2, and determining the treatment response based on the determined expression levels. In an embodiment the ADT is AADT, NADT or SADT, preferably AADT or NADT, more preferably NADT. It is understood that the expression levels of the respective genes may also be detected on the protein level by for example ELISA or immunohistochemistry. In such case the measured or quantified protein levels is assumed to be indicate of the gene expression levels and thus to provide an indirect measurement of the respective gene expression levels. Accordingly in the case of ELISA and immunohistochemistry, for the purpose of the invention antibodies are considered probes for specifically detecting and quantifying the expression level of the respective genes

In a fifth aspect the invention relates to a non-steroidal androgen receptor inhibitor for use in the treatment, prevent or amelioration of prostate cancer in a subject as an androgen deprivation therapy, the use comprising: receiving values representing the expressions levels of the genes GUCY1A1, PLS3, ARSD and CUX2 obtained from a prostate cancer sample of the subject, and determining a treatment response to ADT based on the GUCY1A1, PLS3, ARSD and CUX2 expression levels, wherein: a poor treatment response is predicted when the expression levels of both GUCY1A1 and PLS3 exceed predetermined expression level thresholds, and/or when expression levels of both ARSD and CUX2 exceed predetermined expression level thresholds; and a good treatment response is predicted when the expression levels of GUCY1A1 and PLS3 do both not exceed predetermined expression level thresholds, and the expression levels of ARSD and CUX2 do both not exceed predetermined expression level thresholds, wherein the non-steroidal androgen receptor inhibitor is administered to the subject as a neoadjuvant androgen deprivation therapy when a good treatment response is predicted. In an embodiment the ADT is AADT, NADT or SADT, preferably AADT or NADT, more preferably NADT.

In an embodiment the androgen deprivation therapy comprises treatment with a non-steroidal androgen receptor inhibitor. In an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide. In an embodiment the non-steroidal androgen receptor inhibitor. In an embodiment the non-steroidal androgen receptor inhibitor is Enzalutamide. In an embodiment the androgen deprivation therapy comprises treatment with a non-steroidal androgen receptor inhibitor combined with a different treatment. In an embodiment the androgen deprivation therapy further comprises treatment with one or more of: a CYP17A1 inhibitor, an antigonadotropin, and an glucocorticoid receptor agonist. Non limiting examples of CYP17A1 inhibitors are Abiraterone acetate, Ketoconazole, and Seviteronel. Preferably the CYP17A inhibitor is Abiraterone acetate. Non limiting examples antigonadotropins are Estrogens such as estradiol and its esters, ethinylestradiol, conjugated estrogens, diethylstilbestrol, GnRH analogues such as GnRH agonists (e.g., goserelin, leuprorelin) or GmRH antagonists (e.g., cetrorelix) or Progestogens (e.g., chlormadinone acetate, cyproterone acetate, gestonorone caproate, medroxyprogesterone acetate, megestrol acetate). Preferably the antigonadotropin is a GnRH analogue, more preferably a GnRH agonist, most preferably leuprorelin. Non limiting examples of glucocorticoid receptor agonists are prednisone, Dagrocorat, Fosdagrocorat, and Mapracorat. In a preferred embodiment the glucocorticoid receptor agonist is prednisone. Thus in an embodiment the androgen deprivation therapy further comprises treatment with one or more, for example one, two or all, of Leuprolide, Abiraterone Acetate, and Prednisone.

In a further exemplary example of an AI derived model that can be used to predict the outcome of ADT treatment of a prostate cancer patient, is a model based on the expression levels of GUCY1A1, PLS3, ARSD and CUX2. It was found by the inventor that when either one of the following conditions was met:
- CIAO1 and CSK expression levels are high; and/or
- EZR and INHBA expression levels are high,
the developed model predicts a poor outcome of ADT treatment. In particular, the model predicts a high chance of biochemical recurrence (BCR). When none of the above conditions are met (thus CIAO1 and CSK expression levels are both not high, and EZR and INHBA expression levels are both not high), a good outcome is predicted for ADT treatment. In particular, the model predicts a low chance of BCR. The predictive model is useful as it can be used to provide a treatment recommendation for the patient. For example, when the model predicts a good outcome for ADT, it can be used as a confirmation to provide ADT, alternatively, when a poor outcome of ADT is predicted a different approach may be used, such as an alternative treatment or ADT supplemented with additional treatment.

Accordingly, in a further aspect the invention herein describes a computer implemented method for predicting a treatment response of a subject suffering from prostate cancer to androgen deprivation therapy (ADT), the method comprising: receiving a value representing the expressions levels of the genes CIAO1, CSK, EZR and INHBA obtained from a prostate cancer sample of the subject, and determining the outcome based on the CIAO1, CSK, EZR and INHBA expression levels, wherein: a poor outcome is predicted when the expression levels of both CIAO1 and CSK exceed predetermined expression level thresholds, and/or when expression levels of both EZR and INHBA exceed predetermined expression level thresholds; and a good outcome is predicted when the expression levels of CIAO1 and CSK do both not exceed predetermined expression level thresholds, and the expression levels of EZR and INHBA do both not exceed predetermined expression level thresholds. In an embodiment the outcome is chance of biochemical recurrence. In an embodiment the subject is treated with ADT, preferably NADT, SADT, or AADT, more preferably NADT.

In an embodiment the outcome is biochemical recurrence. Thus a poor outcome indicates a high chance of biochemical recurrence, and a good outcome indicates a low chance of biochemical recurrence.

The method described herein may further include an active step of determining expression levels, therefore in an embodiment the invention relates to a method for predicting a treatment response of a subject suffering from prostate cancer to androgen deprivation therapy (ADT), the method comprising: determining the expressions levels of the genes CIAO1, CSK, EZR and INHBA in a prostate cancer sample obtained from the subject, and determining the treatment response to ADT based on the CIAO1, CSK, EZR and INHBA expression levels, wherein: a poor treatment response is predicted when the expression levels of both CIAO1 and CSK exceed predetermined expression level thresholds, and/or when expression levels of both EZR and INHBA exceed predetermined expression level thresholds; and a good treatment response is predicted when the expression levels of CIAO1 and CSK do both not exceed predetermined expression level thresholds, and the expression levels of EZR and INHBA do both not exceed predetermined expression level thresholds.

The model uses the expression levels of the genes CIAO1, CSK, EZR and INHBA. For each of these expression levels it is determined whether the expression level is high or not high. This may for example be done by setting a threshold expression level, above which the expression level is defined as high, and below which the expression level is defined as not high or low. Such threshold can easily be determined in a reference sample, or preferably a cohort of reference samples. For this the expression level is preferably quantified. Quantification may for example be done by normalizing to a household gene or by calculating reads per million, but other suitable method for quantifying gene expression levels are known in the art and may also be suitably used in the method described herein. Thus when used herein the term "high expression level" or "the expression level is high" can be interpreted as exceeding a threshold expression level for said gene. In this respect it is appreciated that a high expression level will be different for each gene and thus for each gene individually it needs to be determined what constitutes a high expression level, for example by establishing the appropriate threshold above which it is deemed high. The threshold may be determined using methods common in the art. For example from a pool of prostate cancer patients which includes responders and non-responders to ADT expression levels may be collected for the respective gene and the threshold may be set as the mean or average expression level of this pool. Alternatively average expression levels may be determined for responders and non-responder and the threshold may be set as a value in between those average expression level values. The threshold is preferably chosen such as to optimize separation between the responder and non-responder groups.

Thus when used herein a "value representing an expression level" refers to the quantification of said expression level reflected in a value. Such value may for example be expressed as transcripts per million or a normalized expression level. Expression levels may be normalized using one or more household genes. In one realization, the gene expression level for each of CIAO1, CSK, EZR and INHBA is normalized with respect to one or more household genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these household genes.

In an embodiment the expression levels thresholds for each of CIAO1, CSK, EZR and INHBA are predetermined in a cohort of prostate cancer samples from patients having received ADT, for example NADT, SADT, or AADT.

In the present finding it is described that a poor outcome is predicted in case that at least one of the conditions is met that (1) CIAO1 and CSK expression levels are high; and/or
(2) EZR and INHBA expression levels are high. If neither of this condition is met a good outcome is predicted. For example: if CIAO1 and CSK expression levels are high and EZR and INHBA expression levels are low, a poor outcome is predicted because the first criteria is met. If CIAO1 and CSK expression levels are high and EZR and INHBA expression levels are high, a poor outcome is predicted because both the first and the second criteria are met. If CIAO1 and CSK expression levels are low and EZR and INHBA expression levels are high, a poor outcome is predicted because the second criteria is met. If CIAO1 and CSK expression levels are low and EZR and INHBA expression levels are low, a good outcome is predicted because none of the criteria are met. If CIAO1 expression levels are high and CSK, EZR and INHBA expression levels are low, a good outcome is predicted because none of the criteria are met. If COAI1 and INHBA expression levels are high and CSK, and EZR expression levels are low, a good outcome is predicted because none of the criteria are met.

The term "CIAO1" refers to the human Probable cytosolic iron-sulfur protein assembly protein gene (Ensembl: ENSG00000144021), for example, to the sequence as defined in NCBI Reference Sequence NM_004804.3, specifically, to the nucleotide sequence as set forth in SEQ ID NO:9, which correspond to the sequences of the above indicated NCBI Reference Sequences of the CIAO1 transcript, and also relates to the corresponding amino acid sequence for example as set forth in SEQ ID NO: 10, which correspond to the protein sequences defined in NCBI Protein Accession Reference Sequence NP_004795.1 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO: 10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "CSK" refers to the human C-terminal Src kinase gene (Ensembl: ENSG00000103653), for example, to the sequence as defined in NCBI Reference Sequence NM_001127190.2, specifically, to the nucleotide sequence as set forth in SEQ ID NO:11, which correspond to the sequences of the above indicated NCBI Reference Sequences of the CSK transcript, and also relates to the corresponding amino acid sequence for example as set forth in SEQ ID NO:12, which correspond to the protein sequences defined in NCBI Protein Accession Reference Sequence NP_001120662.1 encoding the CSK polypeptide.

The term "CSK" also comprises nucleotide sequences showing a high degree of homology to CSK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or amino acid sequences being at least 75%, 80%, 85%, 90%, 91%, 92%, 93%, 94%, 95%, 96%, 97%, 98% or 99% identical to the sequence as set forth in SEQ ID NO:12 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:12 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11.

The term "EZR" refers to the human Ezrin gene (Ensembl: ENSG00000092820), for example, to the sequence as defined in NCBI Reference Sequence NM_001111077.2, specifically, to the nucleotide sequence as set forth in SEQ ID NO:13, which correspond to the sequences of the above indicated NCBI Reference Sequences of the EZR transcript, and also relates to the corresponding amino acid sequence for example as set forth in SEQ ID NO:14, which correspond to the protein sequences defined in NCBI Protein Accession Reference Sequence NP_001104547.1 encoding the EZR polypeptide.

The term "EZR" also comprises nucleotide sequences showing a high degree of homology to EZR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:13 or amino acid sequences being at least 75%, 80%, 85%, 90%, 91%, 92%, 93%, 94%, 95%, 96%, 97%, 98% or 99% identical to the sequence as set forth in SEQ ID NO:14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:13.

The term "INHBA" refers to the human Inhibin, beta A gene (Ensembl: ENSG00000122641), for example, to the sequence as defined in NCBI Reference Sequence NM_002192.4, specifically, to the nucleotide sequence as set forth in SEQ ID NO:15, which correspond to the sequences of the above indicated NCBI Reference Sequences of the INHBA transcript, and also relates to the corresponding amino acid sequence for example as set forth in SEQ ID NO:16, which correspond to the protein sequences defined in NCBI Protein Accession Reference Sequence NP_002183.1 encoding the INHBA polypeptide.

The term "INHBA" also comprises nucleotide sequences showing a high degree of homology to INHBA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:15 or amino acid sequences being at least 75%, 80%, 85%, 90%, 91%, 92%, 93%, 94%, 95%, 96%, 97%, 98% or 99% identical to the sequence as set forth in SEQ ID NO:16 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:16 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:15.

When used herein for the CIAO1, CSK, EZR and INHBA based model the outcome preferably indicates biochemical recurrence. Thus a good outcome indicates a low chance or absence of BCR, while a poor outcome indicates a high chance of BCR. When used herein, the term biochemical recurrence indicates a rise of prostate-specific antigen (PSA) levels in the blood of a prostate cancer patient after treatment with surgery or radiation. The method may thus predict an increased or high chance of having BCR when the expression levels of CIAO1 and CSK both do not exceed predetermined expression level thresholds, and/or the expression levels of EZR and INHBA both do not exceed predetermined expression level thresholds.

When used herein a poor outcome preferably indicates a high chance of BCR. The method may thus predict an increased or high chance of having BCR when the expression levels of both CIAO1 and CSK exceed predetermined expression level thresholds, and/or when expression levels of both EZR and INHBA exceed predetermined expression level thresholds.

The methods as broadly described herein predict the outcome of androgen deprivation therapy, e.g. AADT, NADT or SADT as further defined and described in detail above. In an embodiment the androgen deprivation therapy comprises treatment with a non-steroidal androgen receptor inhibitor. In an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide. In an embodiment the non-steroidal androgen receptor inhibitor. In an embodiment the non-steroidal androgen receptor inhibitor is Enzalutamide. In an embodiment the androgen deprivation therapy comprises treatment with a non-steroidal androgen receptor inhibitor combined with a different treatment. In an embodiment the androgen deprivation therapy further comprises treatment with one or more of: a CYP17A1 inhibitor, an antigonadotropin, and an glucocorticoid receptor agonist. Non limiting examples of CYP17A1 inhibitors are Abiraterone acetate, Ketoconazole, and Seviteronel. Preferably the CYP17A inhibitor is Abiraterone acetate. Non limiting examples antigonadotropins are Estrogens such as estradiol and its esters, ethinylestradiol, conjugated estrogens, diethylstilbestrol, GnRH analogues such as GnRH agonists (e.g., goserelin, leuprorelin) or GnRH antagonists (e.g., cetrorelix) or Progestogens (e.g., chlormadinone acetate, cyproterone acetate, gestonorone caproate, medroxyprogesterone acetate, megestrol acetate). Preferably the antigonadotropin is a GnRH analogue, more preferably a GnRH agonist, most preferably leuprorelin. Non limiting examples of glucocorticoid receptor agonists are prednisone, Dagrocorat, Fosdagrocorat, and Mapracorat. In a preferred embodiment the glucocorticoid receptor agonist is prednisone. Thus in an embodiment the androgen deprivation therapy further comprises treatment with one or more, for example one, two or all, of Leuprolide, Abiraterone Acetate, and Prednisone.

In an embodiment the method further comprises a step of providing the prediction of the treatment response to the subject or a medical caregiver. In an embodiment the method further comprises a step of providing a recommended treatment strategy to the subject or a medical caregiver. In an embodiment the recommended treatment strategy comprises recommending ADT when a good treatment response is predicted to treatment with ADT. In an embodiment the recommended treatment strategy comprises recommending an alternative treatment or ADT supplemented with additional treatment when a poor treatment response is predicted to treatment with ADT. In an embodiment the alternative treatment consist or comprises of chemotherapy, immunotherapy, targeted therapy or PDE4D7 expression or activity increasing therapy. In an embodiment the additional treatment consist or comprises of chemotherapy, immunotherapy, targeted therapy or PDE4D7 expression or activity increasing therapy. The terms chemotherapy, immunotherapy, targeted therapy or PDE4D7 expression or activity increasing therapy have been defioned above.

In a further aspect the invention relates to a computer program product comprising instructions which, when the program is executed by a computer, cause the computer to carry out a method for predicting a treatment response of a subject suffering from prostate cancer to androgen deprivation therapy (ADT) based on received expression levels for CIAO1, CSK, EZR and INHBA determined in a prostate cancer sample of the subject, wherein the method comprises determining the treatment response based on the received expression levels, wherein a poor treatment response is predicted when the expression levels of both CIAO1 and CSK exceed predetermined expression level thresholds, and/or when expression levels of both EZR and INHBA exceed predetermined expression level thresholds; and a good treatment response is predicted when the expression levels of CIAO1 and CSK do both not exceed predetermined expression level thresholds, and the expression levels of EZR and INHBA do both not exceed predetermined expression level thresholds. In an embodiment the ADT is AADT, NADT or SADT, preferably AADT or NADT, more preferably NADT.

Further disclosed herein is a method of treating, preventing or ameliorating a subject with prostate cancer, the method comprising: predicting a treatment response of a subject suffering from prostate cancer to androgen deprivation therapy (ADT) by receiving values representing the expressions levels of the genes CIAO1, CSK, EZR and INHBA obtained from a prostate cancer sample of the subject, and determining the treatment response to ADT based on the CIAO1, CSK, EZR and INHBA expression levels, wherein: a poor treatment response is predicted when the expression levels of both CIAO1 and CSK exceed predetermined expression level thresholds, and/or when expression levels of both EZR and INHBA exceed predetermined expression level thresholds; and a good treatment response is predicted when the expression levels of CIAO1 and CSK do both not exceed predetermined expression level thresholds, and the expression levels of EZR and INHBA do both not exceed predetermined expression level thresholds; and treating the subject by ADT when a good outcome to ADT is predicted and administering to the subject ADT supplemented with chemotherapy, immunotherapy, targeted therapy or PDE4D7 expression or activity increasing therapy or chemotherapy, immunotherapy, targeted therapy or PDE4D7 expression or activity increasing therapy without ADT when a poor outcome to ADT is predicted. In an embodiment the ADT is AADT, NADT or SADT, preferably AADT or NADT, more preferably NADT.

In an embodiment the ADT, e.g. AADT, NADT or SADT, consists or comprises of administering non-steroidal androgen receptor inhibitor to the subject prior to radical prostatectomy or radiation therapy. In an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide. In an embodiment the ADT consists or comprises of administering non-steroidal androgen receptor inhibitor combined with one or more of: a CYP17A1 inhibitor, an antigonadotropin, and an glucocorticoid receptor agonist to the subject.

In a third aspect the invention relates to a kit of parts comprising primers and/or probes for specifically detecting and quantifying the expression level in biological sample of the genes CIAO1, CSK, EZR and INHBA, preferably wherein the kit is a kit selected from an RNA sequencing kit, a PCR kit, a qPCR kit, a digital PCR kit, an immunohistochemistry kit, an ELISA kit, and an in situ RNA hybridization kit. In an embodiment the kit is for selectively amplifying CIAO1, CSK, EZR and INHBA mRNA in a sample. In an embodiment the kit is for detecting CIAO1, CSK, EZR and INHBA protein in a sample. In an embodiment the kit is for quantifying CIAO1, CSK, EZR and INHBA mRNA or protein. In an embodiment the kit is suitable for use in a method for determining a treatment response to ADT, e.g. AADT, NADT, or SADT.

The kit may further comprise buffers, components or enzymes suitable for the envisioned technique, e.g. RNA sequencing, PCR, qPCR, digital PCR, immunohistochemistry, ELISA, or in situ RNA hybridization. Thus the kit may comprise one or more of a polymerase, a buffer suitable for PCR reactions, dNTP stock solutions, a buffer suitable for RNA sequencing, a primary and/or second antibody for ELISA, a reagent or colorant for detecting ELISA signal, and in situ hybridization buffer.

In a fourth aspect the invention relates to the use of the kit as broadly described herein in determining the treatment response of a subject suffering from prostate cancer to ADT, the use comprising using the primers and/or probes to quantify the expression levels of the genes CIAO1, CSK, EZR and INHBA, and determining the treatment response based on the determined expression levels. In an embodiment the ADT is AADT, NADT or SADT, preferably AADT or NADT, more preferably NADT. It is understood that the expression levels of the respective genes may also be detected on the protein level by for example ELISA or immunohistochemistry. In such case the measured or quantified protein levels is assumed to be indicate of the gene expression levels and thus to provide an indirect measurement of the respective gene expression levels. Accordingly in the case of ELISA and immunohistochemistry, for the purpose of the invention antibodies are considered probes for specifically detecting and quantifying the expression level of the respective genes

In a fifth aspect the invention relates to a non-steroidal androgen receptor inhibitor for use in the treatment, prevent or amelioration of prostate cancer in a subject as an androgen deprivation therapy, the use comprising: receiving values representing the expressions levels of the genes CIAO1, CSK, EZR and INHBA obtained from a prostate cancer sample of the subject, and determining a treatment response to ADT based on the CIAO1, CSK, EZR and INHBA expression levels, wherein: a poor treatment response is predicted when the expression levels of both CIAO1 and CSK exceed predetermined expression level thresholds, and/or when expression levels of both EZR and INHBA exceed predetermined expression level thresholds; and a good treatment response is predicted when the expression levels of CIAO1 and CSK do both not exceed predetermined expression level thresholds, and the expression levels of EZR and INHBA do both not exceed predetermined expression level thresholds, wherein the non-steroidal androgen receptor inhibitor is administered to the subject as a neoadjuvant androgen deprivation therapy when a good treatment response is predicted. In an embodiment the ADT is AADT, NADT or SADT, preferably AADT or NADT, more preferably NADT.

In an embodiment the androgen deprivation therapy comprises treatment with a non-steroidal androgen receptor inhibitor. In an embodiment the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide. In an embodiment the non-steroidal androgen receptor inhibitor. In an embodiment the non-steroidal androgen receptor inhibitor is Enzalutamide. In an embodiment the androgen deprivation therapy comprises treatment with a non-steroidal androgen receptor inhibitor combined with a different treatment. In an embodiment the androgen deprivation therapy further comprises treatment with one or more of: a CYP17A1 inhibitor, an antigonadotropin, and an glucocorticoid receptor agonist. Non limiting examples of CYP17A1 inhibitors are Abiraterone acetate, Ketoconazole, and Seviteronel. Preferably the CYP17A inhibitor is Abiraterone acetate. Non limiting examples antigonadotropins are Estrogens such as estradiol and its esters, ethinylestradiol, conjugated estrogens, diethylstilbestrol, GnRH analogues such as GnRH agonists (e.g., goserelin, leuprorelin) or GmRH antagonists (e.g., cetrorelix) or Progestogens (e.g., chlormadinone acetate, cyproterone acetate, gestonorone caproate, medroxyprogesterone acetate, megestrol acetate). Preferably the antigonadotropin is a GnRH analogue, more preferably a GnRH agonist, most preferably leuprorelin. Non limiting examples of glucocorticoid receptor agonists are prednisone, Dagrocorat, Fosdagrocorat, and Mapracorat. In a preferred embodiment the glucocorticoid receptor agonist is prednisone. Thus in an embodiment the androgen deprivation therapy further comprises treatment with one or more, for example one, two or all, of Leuprolide, Abiraterone Acetate, and Prednisone.

In addition to the two models described in detail above the present disclosure for provides the following models for predicting a high chance of a good response to treatment with ADT, preferably NADT, not occurring in a subject suffering from prostate cancer. The good response preferably is exceptional response as defined above. The features above described for the GUCY1A1, PLS3, ARSD, and CUX2 based model can equally be applied on the below described gene models, as further supported by Example 2:
The expression levels of ARSD and CUX2 are high, and/or the expression levels of ANKRD13A and PLS3 are high;
The expression levels of ANKRD13A and PLS3 are high, and/or the expression level of ARSD is high;
The expression levels of PLS3 and GUCY1A1 are high, and/or the expression level of ARSD is high;
The expression levels of ARSD and CUX2 are high, and/or the expression levels of ANKRD13A and FRY are high;
The expression levels of ARSD and CUX2 are high, and/or the expression levels of ANKRD13A and HLA-DRA are high;
The expression levels of ARSD and CUX2 are high, and/or the expression levels of DDX58 and FNIP2 are high;
The expression levels of ARSD and CUX2 are high, and/or the expression levels of FRY and GUCY1A1 are high;
The expression levels of ARSD and CUX2 are high, and/or the expression levels of TLR8 and GUCY1A1 are high;
The expression levels of SLC25A42 and ARSD are high, and/or the expression levels of ANKRD13A and PLS3 are high;
The expression levels of ARSD and CUX2 are high, and/or the expression level of GUCY1A1 is high;
The expression levels of FRY and GUCY1A1 are high, and/or the expression level of ARSD is high;
The expression levels of ANKRD13A and HLA-DRA are high, and/or the expression level of ARSD is high;
The expression levels of ANKRD13A and GUCY1A1 are high, and/or the expression level of ARSD is high;
The expression levels of ANKRD13A and FRY are high, and/or the expression level of ARSD is high;
The expression levels of TLR8 and GUCY1A1 are high, and/or the expression level of ARSD is high;
The expression level of GUCY1A1 is high, and/or the expression level of ARSD is high;
The expression levels of ARSD and CUX2 are high, and/or the expression levels of IFIH1 and ANKRD13A are high;
The expression levels of ARSD and CUX2 are high, and/or the expression levels of ANKRD13A and TDRD1 are high;
The expression levels of ARSD and CUX2 are high, and/or the expression levels of IFIH1 and TDRD1 are high;
The expression levels of SLC25A42 and ARSD are high, and/or the expression levels of ANKRD13A and FRY are high;
The expression level of ERG is high, and/or the expression levels of PLXNC1 and FYN are high;
The expression levels of SLC25A42 and ARSD are high, and/or the expression levels of ANKRD13A and GUCY1A1 are high;
The expression levels of ANKRD13A and TDRD1 are high, and/or the expression level of ARSD is high;
The expression levels of IFIH1 and ANKRD13A are high, and/or the expression level of ARSD is high;
The expression level of ANKRD13A is high, and/or the expression levels of ARSD are high;
The expression levels of ARSD and CUX are high, and/or the expression levels of CHRM3 and GUCY1A1 are high;
The expression levels of ARSD and CUX2 are high, and/or the expression levels of PLXNC1 and FYN are high;
The expression levels of ARSD and CUX2 are high, and/or the expression levels of HLA-DPA1 and GUCY1A1 are high;
The expression level of ERG is high, and/or the expression levels of ANKRD13A and PLS3 are high;
The expression levels of SLC25A42 and ARSD are high, and/or the expression levels of PLXNC1 and FYN are high;
The expression levels of SLC25A42 and ARSD are high, and/or the expression levels of IFIH1 and ANKRD13A are high;
The expression level of ERG is high, and/or the expression levels of ANKRD13A and FRY are high;
The expression level of ARSD and CUX2 are high, and/or the expression levels of ZNF614 and TDRD1 are high;
The expression level of HLA-DPA1 and GUCY1A1 are high, and/or the expression level of ARSD is high;
The expression level of PLXNC1 and FYN are high, and/or the expression level of ARSD is high;
The expression level of IFIH1 and TDRD1 are high, and/or the expression level of ARSD is high;
The expression level of CHRM3 and GUCY1A1 are high, and/or the expression level of ARSD is high.

For each of the above models individually, if one of the two listed condition are met, or if both of the conditions are met, a poor outcome is predicted. If neither of the conditions are met a good outcome is predicted.

In addition to the two models described in detail above the present disclosure for provides the following models for predicting a high chance biochemical recurrence to treatment with ADT, preferably NADT, in a subject suffering from prostate cancer. The features above described for the CIAO1, CSK, EZR and INHBA based model can equally be applied on the below described gene models, as further supported by Example 4:
The expression levels of CIAO1 and CSK are high, and/or the expression levels of EZR and IGFBP3 are high;
The expression levels of EZR and INHBA are high, and/or the expression level of CIAO1 is high;
The expression levels of EZR and IGFBP3 are high, and/or the expression level of CIAO1 is high;
The expression levels of ANTXR1 and EZR are high, and/or the expression levels of CIAO1 and CSK are high;
The expression levels of ANTXR1 and CIAO1 are high, and/or the expression levels of CIAO1 and CSK are high;
The expression levels of CIAO1 and CSK are high, and/or the expression levels of DRAM1 and IGFBP3 are high;
The expression levels of CIAO1 and CSK are high, and/or the expression levels of DRAM1 and INHBA are high;
The expression levels of ANTXR1 and EZR are high, and/or the expression level of CIAO1 is high;
The expression levels of CIAO1 and CSK are high, and/or the expression levels of IFIT3 and OAS1 are high;
The expression levels of ARHGEF2 and CIAO1 are high, and/or the expression levels of CIAO1 and KCNH8 are high;
The expression levels of MSN and OAS1 are high, and/or the expression levels of CIAO1 and CSK are high;
The expression levels of CIAO1 and CSK are high, and/or the expression levels of CIAO1 and KCNH8 are high;
The expression levels of EZR and IFIT3 are high, and/or the expression levels of CIAO1 and CSK are high;
The expression levels of CIAO1 and CSK are high, and/or the expression levels of EZR and ZFP36L1 are high;
For each of the above models individually, if one of the two listed condition are met, or if both of the conditions are met, a high likelihood of biochemical recurrence is predicted. If neither of the conditions are met a low likelihood of biochemical recurrence is predicted.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

### Examples

Having now generally described the invention, the same will be more readily understood through reference to the following examples which is provided by way of illustration and is not intended to be limiting of the present invention.

### Example 1

The following assessments were recorded for NCT02430480: Prostate-specific antigen (PSA) levels, Cancer grading (Highest Biopsy ISUP Gleason), details of the Medical intervention (Treatment) administered, Reaction evaluation (Response) to the treatment, Non-standard response (Except Response), measurements of Remaining tumor (RCB) in cubic centimeters (cm³), Initial tumor size (Baseline tumor burden) also measured in cm³, adjusted RCB, as well as Immunohistochemistry (IHC) results for ERG and PTEN expressions. Furthermore, the trial tracked ERG and PTEN expression levels and Generic characterization (GC Score).

NCT02430480 and NCT02268175 encompassed the age of participants at diagnosis, the severity of cancer grading from biopsy samples, the site of the biopsy, clinical and pathological staging, initial prostate-specific antigen levels, expressions of ERG and PTEN, treatment responses, mutational burden, as well as genetic scores.

For the two studies the clinical endpoint exceptional response has been used (Tewari et al., 2021; Wilkinson et al., 2021).

### Data analysis

In the context of a diagnostic accuracy study aimed at evaluating signature identification methods, the performance of these models is assessed using the following metrics: accuracy, sensitivity, specificity, the area under the Receiver Operating Characteristic (ROC) curve (AUC), positive predictive value (PPV), and negative predictive value (NPV). These metrics serve as benchmarks for assessing the effectiveness of the models in distinguishing between genuine and forged signatures.

### Data processing

Multiple samples are available for 13 patients of NCT02268175 and NCT02903368. RNA-seq data for these samples are not independent, and this needs to be accounted for in the analysis. In the consolidated dataset, one sample per patient was selected following the methodology of Tewari et al (2021).

A list of 100 genes symbol were pre-selected based on a biological rational including genes from viral-bacterial defense, TCR signaling or correlated with expression of PDE4D7, provided in additional methods. For genes symbols not found in the raw data, aliases of these gene symbol were used instead. Raw gene expression values of the train sets are binned into three discrete categories of equal frequency. For each genes, the quantiles q1 and q2 corresponding to the probabilities of 1/3 and 2/3 respectively are computed. For a given gene, patients with gene expression values higher than q2 were considered to have a high gene expression.

### Association rules and the KEM^{®} platform

Association rules involve identifying patterns in datasets where the presence of a particular set of items (antecedent) is associated with the occurrence of another set of items (consequent). These rules are quantified by metrics such as support, confidence, and lift, providing insights into relationships between variables, commonly applied in market basket analysis and bioinformatics. The KEM^{®} analytical platform and association rules have been described in Hampel et al (2020). Within the KEM^{®} platform, association rules are used as handle to manipulate sets of patients and corresponding sets of related variables. When a set of patients corresponds to a set of variables across a Galois connection, the set of variable is called a Formal Concept: thus, an association rule can always be found in a given Formal Concept. Association rules were mined from the discretized dataset, using the response as consequent and discretized genes expression as antecedent.

### Model Generation

No variable in the original data has enough information to fully explain the endpoint: there is no association that is able to match all the patients with a given endpoint level. KEM^{®} biomarker can be seen as a method to derive new variables that better match the response, in the form of the output of a predictive model. A model consists on a set of association rules, combined together using a decision function, that outputs a binary prediction. In this study, the decision function is a majority vote, with a minimal threshold required for the prediction. Each rule in the model is considered a voter, that predicts the consequent if the antecedents are all verified. Associations are extracted and ranked from the discretized train set and then combined into predictive model using a majority vote. For a given number of rules inside the model and vote threshold for the majority vote, the performance of all possible models is evaluated. Models are then ranked based on their BAC, evaluated on the train set. The model with the highest BAC is selected.

### Cross-validation

The consolidated dataset is split between a train set comprising 70% patients (40) and a test comprising the remaining 30% of patients (18), keeping the same endpoint distribution as the original dataset. A 3-fold crossvalidation strategy is then applied to identify the best hyperparameters of the algorithm. The hyperparameters controlled are the number of rules inside each model, and the threshold used for the majority vote inside the decision function. The set of parameters with the best average performance across folds will be used for the final model evaluation. In this study the set of parameters retained was models consisting of 2 rules combined using an "OR" operator (threshold of 0.5). The same quantiles derived from the train set are used to discretize the raw gene expression data of the test set.

### Final Model selection

The limited number of patient in this study led to an adaptation of the standard, single train-test splitting strategy: the train-test splitting pipeline was repeated 50-times, with different train and test sets each time, thus improving the stability of the retained model. For each iteration, the best model is selected. Genes are then ranked based on the number of times they appear in the selected models. For each model, a score was computed based on the rank of genes inside the model and then used to select the final model retained. Patients in the test set cannot be fully considered as unseen instances, because information from patient in the test is used to compute the score of the model and thus influences the selection. Rather, train-test splitting is effectively used as a form of regularization and not as model validation.

### Continuous score evaluation

From the retained model, a continuous score was derived: for each gene of the model, the probability of a patient to belong in the third tertile of the distribution of gene expression values was modeled based on the continuous gene expression value. This was achieved using a logistic regression, trained on gene expression values of the patients belonging to the original train set. The probabilities are then combined, (see figure 2) to reflect the model combinatorial logic, finally leading to a probability of response for the patient.

### Results

A total of 6,328 models were generated with KEM^{®} Biomarker, ranked based on balanced accuracy on the train set. The top performing KEM^{®} Biomarker model is composed of four genes, ARSD, CUX2, PLS3 and GUCY1A1. This model indicates that a patient is classified as a non-responder if either of the following conditions is met:
1. Both GUCY1A1 and PLS3 are expressed at high levels.
2. Both ARSD and CUX2 are expressed at high levels.

A highly expressed gene refers to a gene for which the level of mRNA produced is relatively high compared to other patients in the same sample population. Specifically, when gene expression values are divided into groups based on their quantile distribution, a gene is considered highly expressed if the expression value for a particular patient falls within the top third of the distribution for that gene. This classification suggests that the gene is more actively transcribed into mRNA and potentially produces a higher abundance of the corresponding protein product in the cells or tissues under investigation. High gene expression levels may indicate increased biological activity or significance of the gene in the context of the studied condition or phenotype.

The four genes found in the model are biologically relevant to cancer: significant up-regulation of ARSD was observed in tumor tissues in comparison with its antisense (p<0.05)(Bahramian et al., 2020). CUX2 was found to have expression levels that were upregulated in papillary thyroid cancer (Sun et al., 2019, p. 2). The α 1 and β 1 subunits (GUCY1A1, GUCY1B1) of soluble guanylyl cyclase (sGC) as major ERG-regulated endothelial genes were found also tightly associated with ERG expression in prostate cancer patient samples (Zhou et al., 2019). PLS3 messenger RNA is overexpressed in PACA tissue compared with normal tissue (Xin et al., 2020, p. 3).

The following table shows the performance of the model, with an overall specificity of 0.92, a sensitivity of 0.87 and a balanced accuracy of 0.87.

**Table 1: Performances of the model**

| BAC | SEN | SPE | PPV | NPV | TP | FP | TN | | FN |
|---|---|---|---|---|---|---|---|---|---|
| Overall | 0.87 | 0.81 | 0.92 | 0.93 | 0.8 | 26 | 2 | 24 | 6 |
| Test | 0.84 | 0.8 | 0.88 | 0.89 | 0.78 | 8 | 1 | 7 | 2 |
| Train | 0.88 | 0.82 | 0.94 | 0.95 | 0.81 | 18 | 1 | 17 | 4 |

**Table 2: Immunoscore performance was obtained based on the Youden index of the Immunoscore (threshold = 0.48). The rest of the KEM^{®} Biomarker models were sent in a separate document.**

| Rank | BAC(train) | Model rules |
|---|---|---|
| 1 | 0.881 | (ARSD{High} AND CUX2{High}) OR (PLS3{High} AND |
| GUCY1A1{High}) | | |
| 1 | 0.881 | (ARSD{High} AND CUX2{High}) OR (ANKRD13A{High} AND |
| PLS3{High}) | | |
| 3 | 0.876 | CHRM3{High} OR CD247{High} |
| 4 | 0.871 | (ANKRD13A{High} AND PLS3{High}) OR ARSD{High} |
| 4 | 0.871 | (PLS3{High} AND GUCY1A1{High} OR ARSD{High} |
| 6 | 0.861 | Immunoscore (reference) |
| 7 | 0.858 | (ARSD{High} AND CUX2{High}) OR (ANKRD13A{High} AND |
| (MAML3{High}) | | |
| 7 | 0.858 | (ARSD{High} AND CUX2{High}) OR (ANKRD13A{ High} AND |
| FRY{High}) | | |
| 7 | 0.858 | (ARSD{High} AND CUX2{High}) OR (ANKRD13A{High} AND |
| HLA-DRA{High}) | | |
| 7 | 0.858 | (ARSD{High} AND CUX2{High}) OR (ANKRD13A{High} AND |
| GUCY1A1 {High}) | | |

**Table 3: parameters of the discrete model (threshold), and of the continuous model (intercept and slope)**

| Gene | Threshold | Intercept β0 | Slope β1 |
|---|---|---|---|
| ARSD | 40.98 | -1.07 | 3.80 |
| CUX2 | 53.35 | -0.80 | 3.71 |
| PLS3 | 173.57 | -1.18 | 3.83 |
| GUCY1A1 | 55.2 | -1.21 | 3.76 |

### Example 2

De method described in Example 1 has resulted in a large number of models.

The models with a BAC training score of 0.8 or higher AND a BAC test score of 0.8 or higher are lsited below:

**Table 4 - alternative models generated with the described method.**

| BAC | BAC | BAC | |
|---|---|---|---|
| train | test | overall | Models |
| 0.881 | 0.838 | 0.868 | ( ARSD{High} & CUX2{High}) OR ( PLS3{High} & GUCY1A1{High} ) |
| 0.881 | 0.9 | 0.887 | ( ARSD{High} & CUX2{High}) OR ( ANKRD13A{High} & PLS3{High}) |
| 0.871 | 0.888 | 0.876 | ( ANKRD13A{High} & PLS3{High}) OR ( ARSD{High} ) |
| 0.871 | 0.825 | 0.857 | ( PLS3{High} & GUCY1A1{High} ) OR ( ARSD{High} ) |
| 0.859 | 0.838 | 0.852 | ( ARSD{High} & CUX2{High} ) OR ( ANKRD13A{High} & FRY{High} ) |
| 0.859 | 0.9 | 0.871 | ( ARSD{High} & CUX2{High} ) OR ( ANKRD13A{High} & HLA-DRA{High}) |
| 0.859 | 0.9 | 0.871 | ( ARSD{High} & CUX2{High}) OR ( DDX58{High} & FNIP2{High}) |
| 0.859 | 0.838 | 0.852 | ( ARSD{High} & CUX2{High} ) OR ( FRY{High} & GUCY1A1{High} ) |
| 0.859 | 0.838 | 0.852 | ( ARSD{High} & CUX2{High} ) OR ( TLR8{High} & GUCY1A1{High} ) |
| 0.854 | 0.85 | 0.852 | ( SLC25A42{High} & ARSD{High}) OR ( ANKRD13A{High} & PLS3{High}) |
| 0.854 | 0.838 | 0.849 | ( ARSD{High} & CUX2{High} ) OR ( GUCY1A1{High} ) |
| 0.848 | 0.825 | 0.841 | ( FRY{High} & GUCY1A1{High}) OR (ARSD{High} ) |
| 0.848 | 0.825 | 0.841 | ( ANKRD13A{High} & HLA-DRA{High}) OR (ARSD{High} ) |
| 0.848 | 0.888 | 0.861 | ( ANKRD13A{High} & GUCY1A1{High}) OR (ARSD{High} ) |
| 0.848 | 0.888 | 0.861 | ( ANKRD13A{High} & FRY{High}) OR ( ARSD{High} ) |
| 0.848 | 0.825 | 0.841 | ( TLR8{High} & GUCY1A1{High}) OR ( ARSD{High} ) |
| 0.843 | 0.825 | 0.838 | ( GUCY1A1{High} ) OR ( ARSD{High} ) |
| 0.836 | 0.9 | 0.856 | ( ARSD{High} & CUX2{High}) OR ( IFIH1{High} & ANKRD13A{High}) |
| 0.836 | 0.838 | 0.837 | ( ARSD{High} & CUX2{High}) OR ( ANKRD13A{High} & TDRD1{High} ) |
| 0.836 | 0.838 | 0.837 | ( ARSD{High} & CUX2{High}) OR ( IFIH1{High} & TDRD1{High} ) |
| 0.831 | 0.85 | 0.837 | ( SLC25A42{High} & ARSD{High}) OR ( ANKRD13A{High} & FRY{High} ) |
| 0.831 | 0.838 | 0.833 | ( ERG{High} ) OR ( PLXNC1{High} & FYN{High} ) |
| | | | ( SLC25A42{High} & ARSD{High}) OR ( ANKRD13A{High} & |
| 0.831 | 0.85 | 0.837 | GUCY1A1{High} ) |
| 0.826 | 0.825 | 0.826 | ( ANKRD13A{High} & TDRD1{High}) OR (ARSD{High} ) |
| 0.826 | 0.888 | 0.845 | ( IFIH1{High} & ANKRD13A{High}) OR (ARSD{High} ) |
| 0.816 | 0.825 | 0.819 | ( ANKRD13A{High}) OR ( ARSD{High} ) |
| 0.813 | 0.85 | 0.825 | ( ARSD{High} & CUX2{High} ) OR ( CHRM3{High} & GUCY1A1{High} ) |
| 0.813 | 0.85 | 0.825 | ( ARSD{High} & CUX2{High} ) OR ( PLXNC1{High} & FYN{High} ) |
| 0.813 | 0.838 | 0.821 | ( ARSD{High} & CUX2{High} ) OR ( HLA-DPA1{High} & GUCY1A1{High} ) |
| 0.808 | 0.838 | 0.817 | ( ERG{High}) OR ( ANKRD13A{High} & PLS3{High}) |
| 0.808 | 0.85 | 0.821 | ( SLC25A42{High} & ARSD{High} ) OR ( PLXNC1{High} & FYN{High} ) |
| 0.808 | 0.85 | 0.821 | ( SLC25A42{High} & ARSD{High}) OR ( IFIH1{High} & ANKRD13A{High}) |
| 0.808 | 0.838 | 0.817 | ( ERG{High}) OR (ANKRD13A{High} & FRY{High}) |
| 0.808 | 0.838 | 0.817 | ( ARSD{High} & CUX2{High}) OR ( ZNF614{High} & TDRD1{High} ) |
| 0.803 | 0.825 | 0.81 | ( HLA-DPA1{High} & GUCY1A1{High}) OR (ARSD{High} ) |
| 0.803 | 0.838 | 0.814 | ( PLXNC1{High} & FYN{High} ) OR ( ARSD{High} ) |
| 0.803 | 0.825 | 0.81 | ( IFIH1{High} & TDRD1{High} ) OR ( ARSD{High} ) |
| 0.803 | 0.838 | 0.814 | ( CHRM3{High} & GUCY1A1{High}) OR ( ARSD{High} ) |

Each of these models pose a viable alternative for the top model selected in example 1 for further avulation.

### Example 3.

### DARANA: Methods and results

### Methods (Darana)

The same methodology was applied to find a predictive model for the BCR endpoint in NCT03297385, with one difference in the data processing step. Original data were log-normalized using a base 2 logarithm and an offset of 1, so we applied the inverse transformation to remove the logarithm. The resulting genes expression values were then used to generate the models. Before applying the model a new cohort, the same transformations steps that were applied to the raw data of study 3 must be applied to the raw data of the new cohort.

### Results (Darana)

A total of 11,325 models were generated with KEM^{®} Biomarker, ranked based on balanced accuracy on the train set. The top performing KEM^{®} Biomarker model is composed of four genes, C1AO1, CSK, EZR and INHBA. This model indicates that a patient is classified as patient without BCR if either of the following conditions is met:
1. Both CIAO1 and CSK are expressed at high levels.
2. Both EZR and INHBA are expressed at high levels.

A highly expressed gene refers to a gene for which the level of mRNA produced is relatively high compared to other genes in the same sample population. Specifically, when gene expression values are divided into groups based on their quantile distribution, a gene is considered highly expressed if the expression value for a particular patient falls within the top third of the distribution for that gene. This classification suggests that the gene is more actively transcribed into mRNA and potentially produces a relatively higher abundance of the corresponding protein product in the cells or tissues under investigation. High gene expression levels may indicate increased biological activity or significance of the gene in the context of the studied condition or phenotype.

Genes found in the model are biologically relevant to cancer. Ezrin (EZR) is involved in cell network regulation by linking the actin cytoskeleton to the cell membranes and by controlling signal transduction by interaction with adhesion molecules and various growth factor receptors (Clucas & Valderrama, 2014). High levels of Ezrin are associated with metastatic behavior in various types of epithelial cancers including prostate cancer (Song et al., 2020). It has been identified as one of the proteins showing increased abundance in prostate cancer tissue, indicating its potential role in prostate cancer development or progression (Vujicic et al., 2022). Expression profiling revealed significant up-regulation of INHBA in BCR patients (Lautert-Dutra et al., 2023). T-cell activation is negatively regulated by C-terminal Src kinase (CSK) (Sridaran et al., 2022) Knockdown of CSK in androgen responsive prostate cancer cell line led to increases in cell proliferation (Yang et al., 2015).

The following figure shows the performance of our model, with a specificity of 0.96, a sensitivity of 0.76 and a balanced accuracy of 0.86.

**Table 5 - Performance of the final model**

| | BAC | SEN | SPE | PPV | NPV | TP | FP | TN | FN |
|---|---|---|---|---|---|---|---|---|---|
| Overall | 0.86 | 0.76 | 0.96 | 0.93 | 0.85 | 13 | 1 | 23 | 4 |
| Test | 0.84 | 0.8 | 0.88 | 0.8 | 0.88 | 4 | 1 | 7 | 1 |
| Train | 0.88 | 0.75 | 1 | 1 | 0.84 | 9 | 0 | 16 | 3 |

**Table 6 - parameters of the discrete model (threshold), and of the continuous model (intercept and slope)**

| Gene | Threshold | Intercept β0 | Slope β1 |
|---|---|---|---|
| CIAO1 | 83.68 | -1.67 | 3.71 |
| CSK | 70.55 | -0.83 | 3.81 |
| EZR | 224.4 | -1.42 | 3.43 |
| INHBA | 32.69 | -1.92 | 3.64 |

### Example 4

The method described in Example 4 further resulted in additional predictive models. Those models with a BAC training score of 0.8 or above AND a BAC test score of 0.8 or above are listed here:

**Table 7 - alternative models using the Darana method**

| BAC | BAC | BAC | Models |
|---|---|---|---|
| train | test | overall | |
| 0.875 | 0.838 | 0.862 | ( CIAO1{High} & CSK{High}) OR ( EZR{High} & INHBA{High}) |
| 0.875 | 0.838 | 0.862 | ( CIAO1{High} & CSK{High} ) OR ( EZR{High} & IGFBP3{High} ) |
| 0.854 | 0.812 | 0.837 | ( EZR{High} & INHBA{High}) OR ( CIAO1{High} ) |
| 0.854 | 0.812 | 0.837 | ( EZR{High} & IGFBP3{High} ) OR ( CIAO1{High} ) |
| 0.833 | 0.838 | 0.832 | ( ANTXR1{High} & EZR{High} ) OR ( CIAO1{High} & CSK{High} ) |
| 0.833 | 0.838 | 0.832 | ( ANTXR1{High} & CIAO1{High} ) OR ( CIAO1{High} & CSK{High} ) |
| 0.812 | 0.838 | 0.82 | ( CIAO1{High} & CSK{High}) OR ( DRAM1{High} & IGFBP3{High}) |
| 0.812 | 0.838 | 0.82 | ( CIAO1{High} & CSK{High}) OR ( DRAM1{High} & INHBA{High}) |
| 0.812 | 0.812 | 0.808 | ( ANTXR1{High} & EZR{High} ) OR ( CIAO1{High} ) |
| 0.802 | 0.838 | 0.811 | ( CIAO1{High} & CSK{High}) OR ( IFIT3{High} & OAS1{High} ) |
| 0.802 | 0.838 | 0.811 | ( ARHGEF2{High} & CIAO1{High} ) OR ( CIAO1{High} & KCNH8{High}) |
| 0.802 | 0.838 | 0.811 | ( MSN{High} & OAS1{High} ) OR ( CIAO1{High} & CSK{High}) |
| 0.802 | 0.838 | 0.811 | ( CIAO1{High} & CSK{High} ) OR ( CIAO1{High} & KCNH8{High} ) |
| 0.802 | 0.838 | 0.811 | ( EZR{High} & IFIT3{High}) OR ( CIAO1{High} & CSK{High}) |
| 0.802 | 0.838 | 0.811 | ( CIAO1{High} & CSK{High} ) OR ( EZR{High} & ZFP36L1{High} ) |

Each of these models pose a viable alternative for the top model selected in example 3 for further avulation.

**Table 8- List of genes and corresponding Ensembl identifiers**

| Gene | ENSEMBL ID | Gene | ENSEMBL ID | Gene | ENSEMBL ID |
|---|---|---|---|---|---|
| ABCC5 | ENSG00000114770 | DOCK11 | ENSG00000147251 | MSN | ENSG00000147065 |
| ABI3BP | ENSG00000154175 | DOCK2 | ENSG00000134516 | MYD88 | ENSG00000172936 |
| ACSL3 | ENSG00000123983 | DRAM1 | ENSG00000136048 | NPNT | ENSG00000168743 |
| ADA2 | ENSG00000093072 | ERG | ENSG00000157554 | OAS1 | ENSG00000089127 |
| AIM2 | ENSG00000163568 | EZR | ENSG00000092820 | OSBPL3 | ENSG00000070882 |
| ANKRD13A | ENSG00000076513 | FNIP2 | ENSG00000052795 | PAG1 | ENSG00000076641 |
| ANTXR1 | ENSG00000169604 | FRMD4A | ENSG00000151474 | PDE3B | ENSG00000152270 |
| APOBEC3A | ENSG00000128383 | FRY | ENSG00000073910 | PDE4D | ENSG00000113448 |
| ARHGEF2 | ENSG00000116584 | FYN | ENSG00000010810 | PLS3 | ENSG00000102024 |
| ARSD | ENSG00000006756 | GJB1 | ENSG00000169562 | PLXNC1 | ENSG00000136040 |
| AUTS2 | ENSG00000158321 | GNB4 | ENSG00000114450 | PRKACA | ENSG00000072062 |
| BCAT1 | ENSG00000060982 | GPRIN3 | ENSG00000185477 | PRKACB | ENSG00000142875 |
| BCL2 | ENSG00000171791 | GUCY1A1 | ENSG00000164116 | PTPRC | ENSG00000081237 |
| CACNA1D | ENSG00000157388 | HLA-DPA1 | ENSG00000231389 | RAB3B | ENSG00000169213 |
| CD2 | ENSG00000116824 | HLA-DRA | ENSG00000204287 | RAP1GAP2 | ENSG00000132359 |
| CD247 | ENSG00000198821 | IFI16 | ENSG00000163565 | SERPING1 | ENSG00000149131 |
| CD28 | ENSG00000178562 | IFIH1 | ENSG00000115267 | SH3KBP1 | ENSG00000147010 |
| CD3E | ENSG00000198851 | IFIT1 | ENSG00000185745 | SLC25A42 | ENSG00000181035 |
| CD3G | ENSG00000160654 | IFIT3 | ENSG00000119917 | SLC39A11 | ENSG00000133195 |
| CD4 | ENSG00000010610 | IGFBP3 | ENSG00000146674 | SPARC | ENSG00000113140 |
| CD74 | ENSG00000019582 | INHBA | ENSG00000122641 | SULF1 | ENSG00000137573 |
| CELF2 | ENSG00000048740 | ITGA4 | ENSG00000115232 | SYK | ENSG00000165025 |
| CHRM3 | ENSG00000133019 | KCNH8 | ENSG00000183960 | TDRD1 | ENSG00000095627 |
| CIAOI | ENSG00000144021 | KIAA1324 | ENSG00000116299 | THBS2 | ENSG00000186340 |
| CIITA | ENSG00000179583 | KIAA1549 | ENSG00000122778 | TLR8 | ENSG00000101916 |
| COL8A1 | ENSG00000144810 | KIF13B | ENSG00000197892 | VWA2 | ENSG00000165816 |
| CSGALNACT1 | ENSG00000147408 | LAPTM5 | ENSG00000162511 | WIPF1 | ENSG00000115935 |
| CSK | ENSG00000103653 | LAT | ENSG00000213658 | ZAP70 | ENSG00000115085 |
| CTSB | ENSG00000164733 | LCK | ENSG00000182866 | ZBP1 | ENSG00000124256 |
| CUX2 | ENSG00000111249 | LRRFIP1 | ENSG00000124831 | ZFP36L1 | ENSG00000185650 |
| CXCR4 | ENSG00000121966 | LTBP2 | ENSG00000119681 | ZNF614 | ENSG00000142556 |
| CYBB | ENSG00000165168 | MAML3 | ENSG00000196782 | ZNF875 | ENSG00000181666 |
| DDX58 | ENSG00000107201 | MAP7 | ENSG00000135525 | | |
| DHX9 | ENSG00000135829 | MFAP4 | ENSG00000166482 | | |

## Claims

1. A computer implemented method for predicting a treatment response of a subject suffering from prostate cancer to androgen deprivation therapy (ADT), the method comprising:
receiving values representing the expressions levels of the genes GUCY1A1, PLS3, ARSD and CUX2 obtained from a prostate cancer sample of the subject, and
determining the treatment response to ADT based on the GUCY1A1, PLS3, ARSD and CUX2 expression levels, wherein:
a poor treatment response is predicted when the expression levels of both GUCY1A1 and PLS3 exceed predetermined expression level thresholds, and/or when expression levels of both ARSD and CUX2 exceed predetermined expression level thresholds; and
a good treatment response is predicted when the expression levels of GUCY1A1 and PLS3 do both not exceed predetermined expression level thresholds, and the expression levels of ARSD and CUX2 do both not exceed predetermined expression level thresholds.

2. The computer implemented method according to claim 1, wherein the expression levels thresholds for each of GUCY1A1, PLS3, ARSD, and CUX2 are predetermined in a cohort of prostate cancer samples from patients having received ADT.

3. The computer implemented method according to claim 1 or 2, wherein the neoadjuvant androgen deprivation therapy comprises treatment with a non-steroidal androgen receptor inhibitor.

4. The computer implemented method according to claim 3, wherein the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide.

5. The computer implemented method according to claim 3 or 4, wherein the neoadjuvant androgen deprivation therapy further comprises treatment with one or more of: a CYP17A1 inhibitor, an antigonadotropin, and an glucocorticoid receptor agonist.

6. The computer implemented invention according to any one of the preceding claims, wherein the method further comprises a step of providing the prediction of the treatment response to the subject or a medical caregiver.

7. The computer implemented invention according to any one of the preceding claims, wherein the method further comprises a step of providing a recommended treatment strategy to the subject or a medical caregiver.

8. The computer implemented invention according to claim 7, wherein the recommended treatment strategy comprises recommending ADT when a good treatment response is predicted to treatment with ADT, or wherein the recommended treatment strategy comprises recommending ADT supplemented with a supplementary therapy or the recommended treatment strategy comprises an alternative therapy to ADT when a poor treatment response is predicted to treatment with ADT, preferably wherein the supplementary therapy or the alternative therapy is selected from chemotherapy, immunotherapy, targeted therapy or PDE4D7 expression or activity increasing therapy.

9. The computer implemented invention according to any one of the preceding claims, wherein the ADT is NADT, AADT, or SADT.

10. A computer program product comprising instructions which, when the program is executed by a computer, cause the computer to carry out a method for predicting a treatment response of a subject suffering from prostate cancer to neoadjuvant androgen deprivation therapy (NADT) based on received expression levels for GUCY1A1, PLS3, ARSD and CUX2 determined in a prostate cancer sample of the subject, wherein the method comprises
determining the treatment response based on the received expression levels, wherein
a poor treatment response is predicted when the expression levels of both GUCY1A1 and PLS3 exceed predetermined expression level thresholds, and/or when expression levels of both ARSD and CUX2 exceed predetermined expression level thresholds; and
a good treatment response is predicted when the expression levels of GUCY1A1 and PLS3 do both not exceed predetermined expression level thresholds, and the expression levels of ARSD and CUX2 do both not exceed predetermined expression level thresholds.

11. A kit of parts comprising primers and/or probes for specifically detecting and quantifying the expression level in biological sample of the genes GUCY1A1, PLS3, ARSD, and CUX2, preferably wherein the kit is a kit selected from an RNA sequencing kit, a PCR kit, a qPCR kit, a digital PCR kit, an immunohistochemistry kit, an ELISA kit, and an in situ RNA hybridization kit.

12. Use of the kit according to claim 11 in determining the treatment response of a subject suffering from prostate cancer to NADT, the use comprising
using the primers and/or probes to quantify the expression levels of the genes GUCY1A1, PLS3, ARSD, and CUX2, and
determining the treatment response based on the determined expression levels.

13. A non-steroidal androgen receptor inhibitor for use in the treatment, prevent or amelioration of prostate cancer in a subject as a neoadjuvant androgen deprivation therapy, the use comprising:
receiving values representing the expressions levels of the genes GUCY1A1, PLS3, ARSD and CUX2 obtained from a prostate cancer sample of the subject, and
determining a treatment response to NADT based on the GUCY1A1, PLS3, ARSD and CUX2 expression levels, wherein:
a poor treatment response is predicted when the expression levels of both GUCY1A1 and PLS3 exceed predetermined expression level thresholds, and/or when expression levels of both ARSD and CUX2 exceed predetermined expression level thresholds; and
a good treatment response is predicted when the expression levels of GUCY1A1 and PLS3 do both not exceed predetermined expression level thresholds, and the expression levels of ARSD and CUX2 do both not exceed predetermined expression level thresholds,
wherein the non-steroidal androgen receptor inhibitor is administered to the subject as a neoadjuvant androgen deprivation therapy when a good treatment response is predicted.

14. Non-steroidal androgen receptor inhibitor for use according to claim 13, wherein the non-steroidal androgen receptor inhibitor is selected from Flutamide, Nilutamide, Bicalutamide, Enzalutamide, Clascoterone, Apalutamide, Proxalutamide, Cimetidine, Topilutamide, and Darolutamide.

15. Non-steroidal androgen receptor inhibitor for use according to claim 13 or 14, wherein the neoadjuvant androgen deprivation therapy further comprises treatment with one or more of: a CYP17A1 inhibitor, an antigonadotropin, and an glucocorticoid receptor agonist.
